# EUROPEAN PATENT APPLICATION

(11) **EP 4 837 899 A1**
(43) Date of publication of application: **11.11.2026**
(21) Application number: 25174904.0
(22) Date of filing: 07.05.2025
(51) Int. Cl.: C07D 207/48, C07C 253/30, C07C 255/50, C07C 255/51, C07C 255/52, C07C 303/02, C07C 303/38, C07C 309/46, C07C 309/88, C07D 211/96, C07D 239/42, C07D 239/52, C07D 401/04, C07D 401/12, C07D 407/12, C07D 409/12, C07D 493/04

(54) **PROCESS FOR THE IRON-MEDIATED NITRATE REDUCTION FOR THE FUNCTIONALIZATION OF ANILINES AND AMINOHETEROCYCLES**

(71) Applicant: Studiengesellschaft Kohle gGmbH, 45470 Mülheim (DE)
(72) Inventor: RITTER, Tobias, 45470 Mülheim an der Ruhr (DE); SCHULTE, Tim, 45470 Mülheim an der Ruhr (DE); BEHERA, Deepak Kumar, 45470 Mülheim an der Ruhr (DE)

(57) **Abstract**

The present invention refers to a process for the functionalization such as a deaminative chlorosulfonylation, deaminative fluorination, or deaminative cyanation of an aromatic or heteroaromatic amino compound. With the inventive process, the in-situ nitrate reduction allows the direct functionalization of anilines and aminoheterocycles via an aryldiazonium or heteroaryl diazonium salt as a fleeting intermediate in solution.

## Description

The present invention refers to a process for the functionalization such as a deaminative chlorosulfonylation, deaminative fluorination or deaminative cyanation of an aromatic or heteroaromatic primary amino compound. With the inventive process, the iron-mediated in-situ nitrate reduction allows the direct functionalization of anilines and aminoheterocycles via an aryldiazonium or heteroaryl diazonium salt as a fleeting intermediate in solution.

Methods for the halogenation of aromatic cycles including deaminative halogenations have been reported before.

For example, DHERANGE BALU D. ET AL: "Direct Deaminative Functionalization", JOURNAL OF THE AMERICAN CHEMICAL SOCIETY, vol. 145, no. 1 , 22 December 2022 (2022-12-22), pages 17-24, describe a method for the deaminative halogenation of amines. However, the reaction proceeds by isodiazene intermediates and not by diazonium salts. The isodiazene intermediates possess completely different chemical reactivity than diazonium salts. Therefore, the chemical transformations that can be achieved by the method disclosed in said reference are very different from the present process.

Furthermore, GHIAZZA CLEMENT ET AL: "Deaminative chlorination of aminoheterocycles", NATURE CHEMISTRY, vol. 14, no. 1 , 16 December 2021 (2021-12-16), pages 78-84 disclose a method for the deaminative chlorination of heteroaromatic amines. However, the reaction proceeds by pyridinium salts as intermediates, which again have completely different reactivity to diazonium salts. Moreover, the protocol only shows that deaminative chlorination, bromination, and fluorination, iodination reactions are not included.

EP 2 602 242 A1 describes the deaminative functionalization of anilines via in situ generated diazonium salts with TEMPO as initiator for the diazotization. Although the process features the in situ generation of the diazonium salt, the diazotization is still carried out with nitrite-based reagents. Diazotization with nitrite reagents is very fast. Therefore the process still holds the risk of diazonium accumulation. It can therefore be not rendered as safer as in the case of our protocol. Moreover TEMPO is an unstable and expensive reagent which leads to much less utility than our protocol. The main feature of our protocol is the combination of slow nitrate reduction with diazotization and further functionalization. The rate-limiting nitrate reduction prevents the accumulation of diazonium salts, which is novel compared to the patent D3.

However, the prior art has not used rate-limiting nitrate salt or nitrate ester reduction for the in situ generation of diazonium salts in a large scope.

Aryldiazonium salts have become a staple in organic synthesis since their discovery in 1858; their synthesis still follows the protocol developed in the 19^{th} century. Due to the favorable reactivity that often cannot be achieved with other aryl(pseudo)halides, diazonium chemistry continues to grow. Facile extrusion of dinitrogen contributes to the desired reactivity but is also reason for safety concerns. Explosions have occurred since their discovery and still result in accidents with casualties that deter from the utility of aryldiazonium salts. Here we report a diazonium chemistry paradigm shift based on nitrate reduction that avoids diazonium accumulation. Because nitrate reduction is rate-limiting, aryldiazoniums are produced as fleeting intermediates, which results in a safer and often more efficient deaminative halogenation in a single step from anilines.

In the prior art, several attempts of using diazonium salts have been made. Sandmeyer used aryldiazonium salts 140 years ago to achieve the oxidative addition to *d*¹⁰ metals for the synthesis of aryl halides. The reactivity of diazonium salts often surpasses that of other (pseudo)halides and is based on their high electrophilicity, low reduction potential of - 0.16 V versus (vs) standard calomel electrode (SCE), and formation of dinitrogen as a leaving group that enables challenging transformations such as single electron transfer-based oxidative addition to main group elements. Therefore, diazonium salts are still prepared routinely both on laboratory scale and in industrial plants; and arylhalides are still commonly prepared from aryldiazonium salts.

Since the first report on aryldiazonium salt synthesis with nitrous acid, little has changed in their preparation. Nitrosonium (NO⁺) ions, formed through acidic degradation of nitrite-based reagents such as sodium- (NaNO₂) or isoamylnitrite, react with anilines at low temperature, which is commonly required due to the instability of aryldiazonium salts above 5 °C. The resulting salts are either isolated, or accumulate for subsequent treatment with nucleophiles, sometimes at higher temperatures, in a separate second step. Reaction enthalpies of up to ΔH= - 84 kcal mol⁻¹ are common for diazonium decomposition reactions and can result in violent degradation. The often unpredictable stability of aryldiazonium salts can result in detonation upon contact with air and even in solution, which has led to a multitude of severe accidents in undergraduate lab courses, academic research, and industry, with fatal consequences at times. The persistent assumption in the community that the tetrafluoroborate (BF₄⁻) counterion prevents explosions of diazonium salts has been proven to be incorrect since BF₄⁻ salts have also been reported to explode.

To attenuate the risks associated with aryldiazonium batch processes, continuous flow set ups can be employed. Although several large scale processes are in place, such as the Balz-Schiemann reaction for the synthesis of fluorobenzene, small changes in the protocol, small quantities of impurities, or mechanical force can result in unexpected detonations, and every new aryldiazonium salt should be considered dangerous and explosive until proven otherwise. To mitigate the dangers associated with diazonium chemistry, alternative deamination protocols have been developed that, for example, employ pyridines as leaving groups or isodiazenes as intermediates, yet, those reactions typically do not achieve the broad utility and high atom economy of the parent diazotization methods.

Thus, there is the need to provide a process for functionalization such as sulfonylation, fluorination, or cyanation of an aromatic or heteroaromatic amino compound without the danger of using accumulated explosive diazonium compounds.

The problem of the present invention is solved by the provision of the inventive process as defined in the claims. In more detail, the present invention refers to a process for the functionalization of an aromatic or heteroaromatic primary amino compound wherein an aromatic or heteroaromatic primary amino compound is reacted with a compound pair of Fe(lll) nitrate and a sulfur oxygen reductant or mixtures thereof, whereby an aromatic or heteroaromatic-N₂⁺-intermediate compound is provided, which is in situ further subjected to a reaction step selected from a sulfochlorination step, a fluorination step or a cyanation step.

Preferably, the sulfur-oxygen reductant is selected from alkali or ammonium salts of thiosulfuric acid, thiosulfates, polythionates, dithionates, trithionates, tetrathionates, dithionites, sulfites, bisulfites, sulfur dioxide, or metabisulfites, or mixtures thereof.

In an embodiment of the invention, the aromatic or heteroaromatiic primary amino compound is selected from C5 to C20 aryl or heteroaryl primary amino compounds.

In a further embodiment, off the process for the functionalization of an aromatic or heteroaromatic primary amino compound, the reaction is carried out in an optionally aqueous polar aprotic solvent such as, but not limited to: acetonitrile, butyronitrile, ethylacetate, chloroform, 1,2-dichloroethane, dichloromethane, acetone, or mixtures thereof.

In the inventive process, said aromatic or heteroaromatic-N₂⁺-intermediate compound may be further reacted in a sulfochlorination step with SO₂ in the presence of a Cu(I) or Cu (II) salt or complex and a chloride source whereby a chlorosulfonylated aromatic or heteroaromatic compound is obtained. The copper (I) or (II) salt or complex may be CuCl, or CuCl₂. The chloride source may be an alkali metal or metal salt, or a mineral acid, such as HCl.

In the inventive process, said aromatic or heteroaromatic-N₂⁺-intermediate compound may be further reacted in a fluorination step with a fluorination agent whereby a fluorinated aromatic or heteroaromatic compound is obtained. Said fluorination agent may be selected from metal salts such as a silver, an ammonium or an alkali salt of tetrafluoroboric acid, hexafluorophosphoric acid or hexafluoroantimonic acid.

In the inventive process, said aromatic or heteroaromatic-N₂⁺-intermediate compound may also be further reacted in a cyanation step with a Cu(I) or Cu (II) salt or complex and a cyanide source whereby a cyanated aromatic or heteroaromatic compound is obtained. The copper (I) or (II) salt or complex may be CuCN. The cyanation agent may be selected from metal cyanides such as alkali cyanides.

As detailed throughout the present application the present invention allows the functionalisation of an aromatic or heteroaromatic primary amino compound by providing the intermediate diazonium compound by using compound pair of Fe(lll) nitrate and a sulfur oxygen reductant or mixtures thereof so that the formed intermediate compound can be further provided with substituents.

In said compound pair, the sulfur-oxygen reductant may be selected from alkali or ammonium salts of thiosulfuric acid, thiosulfates, polythionates, dithionates, trithionates, tetrathionates, dithionites, sulfites, bisulfites, sulfur dioxide, or metabisulfites, or mixtures thereof.

The inventive method is preferably carried out at room temperature up to an elevated temperature, optionally at the boiling point of the solvent, usually in the range of 20°C to 120°C. The polar organic solvent is not particularly critical and is preferably selected from the group consisting of polar aprotic solvents such as, but not limited to: acetonitrile, butyronitrile, ethylacetate, chloroform, 1,2-dichloroethane, dichloromethane, acetone, or mixtures thereof, optionally in combination with water up to 50 vol.-% of the liquid phase.

For the inventive method, the process can be carried out under an ambient atmosphere or in a closed container. The process can be carried out under a protective atmosphere such as nitrogen, argon or mixtures thereof, but there is no need in view of the reactions partners.

In the context of the aspects of the present invention, the following definitions are more general terms which are used throughout the present application.

When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

The term "aliphatic" includes both saturated and unsaturated, straight chain (*i.e.,* unbranched), branched, acyclic, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, the term "alkyl" includes straight, branched and acyclic alkyl groups. An analogous convention applies to other generic terms such as "alkenyl", "alkynyl", and the like. Furthermore, the terms "alkyl", "alkenyl", "alkynyl", and the like encompass both substituted and unsubstituted groups. In certain embodiments, "lower alkyl" is used to indicate those alkyl groups (acyclic, substituted, unsubstituted, branched or unbranched) having 1-6 carbon atoms.

As used herein, "alkyl" refers to a radical of a straight-chain, branched or cyclic saturated hydrocarbon group having from 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently unsubstituted (an "unsubstituted alkyl") or substituted (a "substituted alkyl") with one or more substituents. In certain embodiments, the alkyl group is an unsubstituted C₁₋₁₀ alkyl (*e.g.*, -CH₃). In certain embodiments, the alkyl group is a substituted C₁₋₁₀ alkyl.

In the inventive process, any aromatic or heteroaromatic primary amino compound having 5 to 30 carbon atoms, optionally having side chains with up to 20 carbon atoms, and optionally up to three heteroatoms selected from O, S or N in the ring structure can be used as starting material, said ring structure having at least one primary amino substituent group on the aromatic or heteroaromatic ring system which primary amino substituent group can be reacted to a diazo group and subsequently replaced by a halogen. Said halogen can be any of Cl, Br or I and may be simply added, depending on the halogen, to the solution in the form of an alkali or ammonium salt, as halogen, or as an halogen alkyl compound such as 1,2-dichloroethane, 1,2-dibromoethane or 1,2-diiodoethane.

Said aromatic or aryl refers to a radical of a monocyclic or polycyclic (*e.g*., bicyclic or tricyclic) 4n+2 aromatic ring system (*e.g*., having 6, 10, or 14 pi electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; *e.g.,* phenyl). In some embodiments, an aryl group has ten ring carbon atoms ("C₁₀ aryl"; *e.g.,* naphthyl such as 1-naphthyl and 2-naphthyl). In some embodiments, an aryl group has fourteen ring carbon atoms ("C₁₄ aryl"; *e.g*., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

"Aralkyl" is a subset of alkyl and aryl and refers to an optionally substituted alkyl group substituted by an optionally substituted aryl group. In certain embodiments, the aralkyl is optionally substituted benzyl. In certain embodiments, the aralkyl is benzyl. In certain embodiments, the aralkyl is optionally substituted phenethyl. In certain embodiments, the aralkyl is phenethyl.

"Heteroaryl" preferably refers to a radical of a 5-14 membered monocyclic or bicyclic 4n+2 aromatic ring system (*e.g*., having 6 or 10 pi electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e.g.*, indolyl, quinolinyl, carbazolyl, and the like) the point of attachment can be on either ring, *i.e.,* either the ring bearing a heteroatom (*e.g.,* 2-indolyl) or the ring that does not contain a heteroatom (*e.g.*, 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

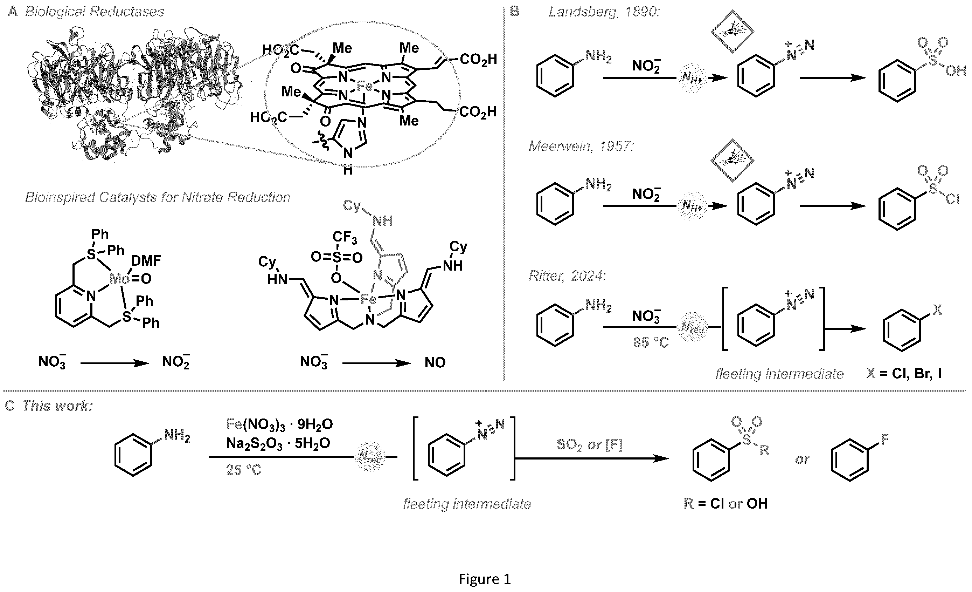

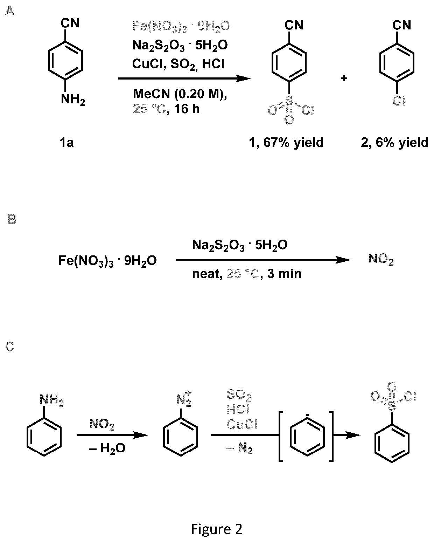

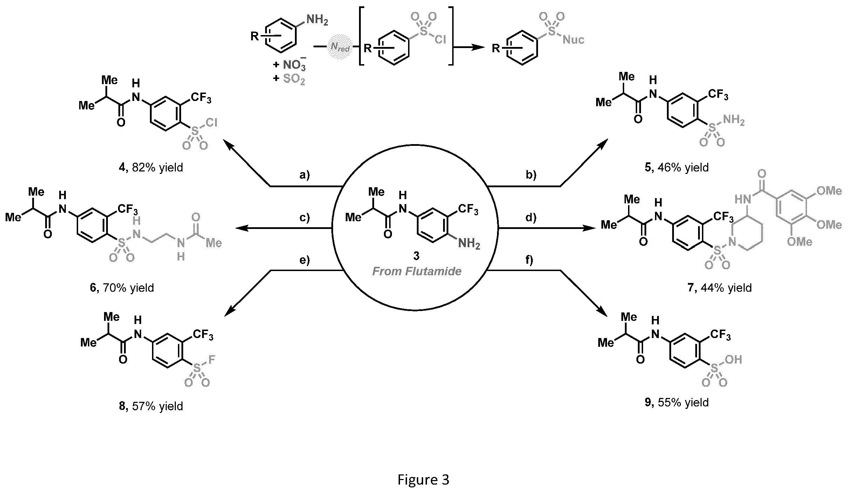

The present invention is further illustrated by the attached Figures and Examples. In the Figures, it is shown:
Fig. 1
   (A) Structure of cd1 nitrite reductase NirS (PDB = 6TSI) with structure of heme d1; Structures of bioinspired catalysts for nitrate reduction.
   (B) Conversion of diazonium salts presented by Landsberg and Meerwein and deaminative halogenation at 85 °C by Ritter.
   (C) This work; direct deaminative sulfonylation and fluorination enabled by iron nitrate reduction at 25 °C
Figure 2 - Mechanism of the deaminative chlorosulfonylation.
   (A) Deaminative chlorosulfonylation with iron nitrate at 25 °C; yields determined by ¹H NMR spectroscopy
   (B) Nitrate reduction with thiosulfate at 23 °C, detection of NO₂ by gas phase IR spectroscopy.
   (C) Mechanistic overview for deaminative sulfonylation; Transition state of nitrate reduction
Fig. 3 - Different transformations for diversification after deaminative sulfonylation Standard conditions (SC): 0.500 mmol reduced flutamide 1, 0.500 mmol Na₂S₂O₃·5H₂O, 0.500 mmol Fe(NO₃)₃ · 9H₂O, 0.500 mmol CuCl, 2.5 mL MeCN, 1.0 mmol HCl (4.0 M in 1,4-dioxane), 5.00 mmol SO₂ (3.00 M in MeCN) at 40 °C for 16 h; a) SC; b) SC, then 2.00 mmol NH₃ (0.50 M in 1,4-dioxane) at 23 °C for 1 h; c) SC, then 2.00 mmol *N-*acetylethylenediamine at 23 °C for 1 h; d) SC, then 0.500 mmol Troxipid and 0.500 mmol K₂CO₃ at 23 °C for 2 h, e) SC, then 1.00 mmol CsF at 23 °C for 1 h; f) SC, then 10 equiv. H₂O and 2.0 equiv. K₂CO₃ at 85 °C for 16 h

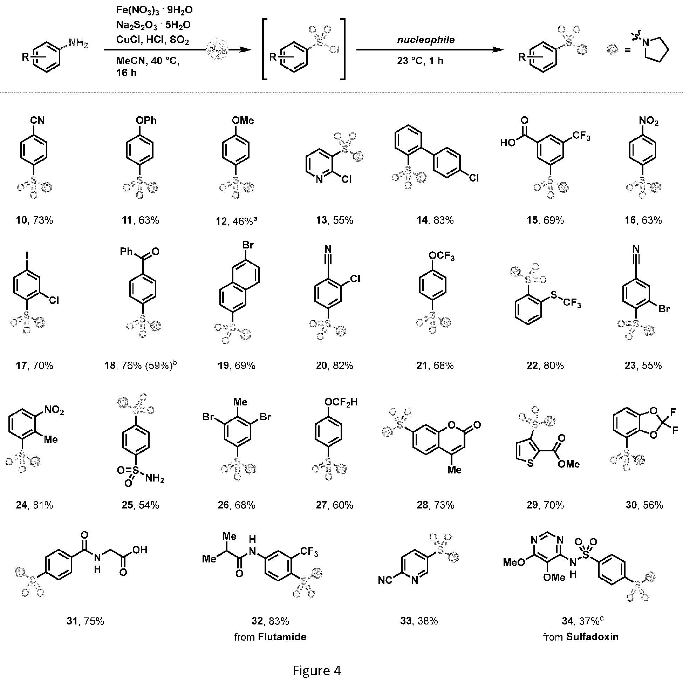

Fig. 4 - Aniline scope for the deaminative chlorosulfonylation.
   Products isolated as sulfonamides with pyrrolidine as nucleophile. Reaction conditions = 0.500 mmol aniline/amino heterocycle, 0.500 mmol Na₂S₂O₃·5H₂O, 0.500 mmol Fe(NO₃)₃ · 9H₂O, 0.500 mmol CuCl, 2.5 mL MeCN, 1.0 mmol HCl (4.0 M in 1,4-dioxane), 5.00 mmol SO₂ (3.00 M in MeCN) at 40 °C for 16 h; then 2.00 mmol pyrrolidine at 23 °C for 1 h. ^{a} isolated on 0.1 mmol scale. ^{b} isolated on 10 mmol scale. ^{c} isolated as sulfonyl chloride.

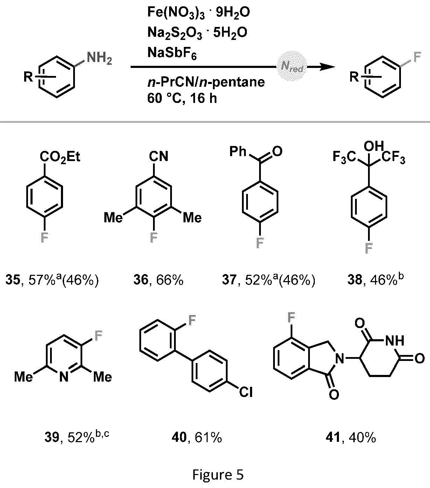

Fig. 5 - Aniline scope for the direct deaminative fluorination.
   Reaction conditions = 0.500 mmol aniline/amino heterocycle, 0.600 mmol Na₂S₂O₃·5H₂O, 1.50 mmol NaSbF₆, 1.00 mmol Fe(NO₃)₃ · 9H₂O, 0.625 mL n-PrCN, 0.625 mL n-pentane, at 60 °C for 16 h (a) isolated on 0.1 mmol scale (b) yield determined by ¹H NMR spectroscopy due to the volatility of compound (c) AgSbF₆ used instead of NaSbF₆.

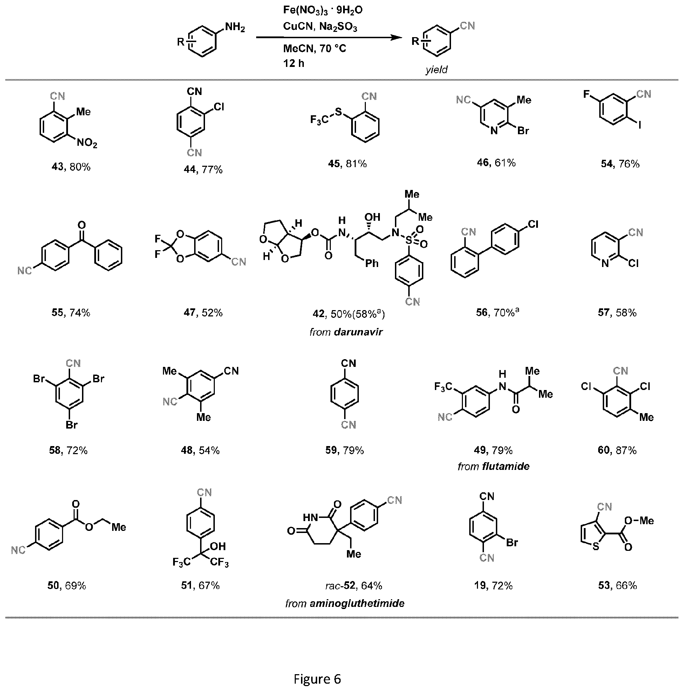

Figure 6 - Scope of anilines and amino heterocycles for the deaminative cyanation.
   Reaction condition is as follows: 0.500 mmol aniline, 1.00 mmol Fe(NO₃)₃·9H₂O, 1.00 mmol CuCN, 0.600 mmol Na₂SO₃, 1.25 ml of acetonitrile, 70 °C, 12 hours. (^{a}) 0.500 mmol aniline, 1.00 mmol Fe(NO₃)₃·9H₂O, 1.00 mmol CuCN, 0.600 mmol Na₂S₂O₄, 1.25 ml of butyronitrile, 70 °C, 12 hours. Supplementary materials provide detailed experimental procedures

The inventors started their work with deliberations concerning a nitrate reduction strategy. According to said considerations, the formation of NO₂⁺ in acidic conditions and the kinetic stability of NO₃⁻ may be the reasons that nitrate salts and their esters have been ignored as diazotization reagents over more than a century. The conversion of NO₂⁻ to NO⁺, and of NO₃⁻ to NO₂⁺ with acid are redox neutral processes. Use of nitrate (N oxidation state = +V) as diazotization reagent therefore requires a reductive pathway in the absence of strong acid for the generation of NO⁺ (N oxidation state = +III). Single electron reduction to nitrogen dioxide (NO₂, N oxidation state = +IV) allows the formation of NO⁺ through dimerization and then disproportionation of dinitrogen tetroxide (N₂O₄). The inventor's protocol relies on sulfur-oxygen reductants which may be selected from alkali or ammonium salts of thiosulfuric acid, thiosulfates, polythionates, dithionates, trithionates, tetrathionates, dithionites, sulfites, bisulfites, sulfur dioxide, or metabisulfites, or mixtures thereof as electron donors for nitrate reduction and can employ readily available iron (III) nitrate. Although NO₃⁻ is thermodynamically strong enough to oxidize nucleophiles like bromide and iodide based on reduction potentials, it is compatible with various nucleophiles in the same reaction mixture, including bromide, iodide, and thiosulfate. In contrast, the hitherto used nitrite and alkylnitrites are incompatible with several nucleophiles because they react by kinetically facile oxidation. Therefore, such nucleophiles cannot be present during diazotization with nitrites; and diazoniums accumulate or must be isolated, which results in the safety issues of global diazonium chemistry.

Because the process of nitrate reduction is the rate-limiting step in the inventor's strategy, diazoniums are produced as fleeting intermediates in only minute concentrations, which results in several fundamental advantages when compared to the conventional diazonium chemistry employed over the past 150 years: i) exothermic decomposition and fast nitrogen evolution of aryldiazoniums is avoided, ii) anilines are used as starting materials directly, which avoids a two-step sequence with isolation or storage of diazonium salts, iii) most aryldiazoniums are unstable above 5 °C, therefore, conventional reactions are generally restricted to low temperature reactions, yet, the inventor's protocol is not limited by such a constraint and may access reaction chemistry beyond traditional diazonium reactions, and iv) nitrate and its esters are more readily available and less expensive than alkylnitrites. In addition, diazotization and functionalization of aryldiazoniums produce H₂O and N₂ as byproducts, as compared to other, modern deaminative strategies with pyridines, carboxylates, or esters as side products.

As shown by the present invention, nitrate reduction with iron nitrate at 25 °C allows to lower the overall reaction temperature and therefore to increase the amount of dissolved SO₂. When the deaminative chlorosulfonylation was carried out with iron nitrate and thiosulfate at 25 °C, 67% of the desired sulfonyl chloride 1 was obtained, with only 6% of concomitant aryl chloride 2 (Fig. 2, A). The inventors understand that the deaminative chlorosulfonylation proceeds via aryldiazonium salts as fleeting intermediates (Fig. 2, C). NO₂ formed via the nitrate reduction process can dimerize to form N₂O₄, which subsequently disproportionates to generate nitrosonium cation (NO⁺) and nitrate, as previously described. Diazonium salts are then generated by reaction of aniline with NO⁺ following the conventional diazotization mechanism. The aryldiazonium reacts with CuCl, SO₂ and HCl to form the sulfonyl chloride via initial formation of an aryl radical under the release of dinitrogen. When the deaminative chlorosulfonylation was carried out with 2-(allyloxy)aniline as radical trap, the respective radical cyclization product was detected, which is consistent with a radical mechanism operating. Additionally, control experiments were carried out in the absence of CuCl or HCl, which resulted in significantly lower yields of sulfonyl chloride when 4-aminobenzonitrile was used as substrate (21% yield without CuCl, 46% yield without HCl, Nitrate reduction can thus be utilized for the direct generation of aryl radicals from anilines.

Reaction optimization with 4-aminobenzonitrile **1a** revealed that a combination of 1.0 equiv. iron nitrate with 1.0 equiv. Na₂S₂O₃·5H₂O in the presence of 1.0 equiv. CuCl, and 2.0 equiv. HCl in 1,4-dioxane with an excess of SO₂ (10 equiv.) in MeCN may lead to the formation of sulfonyl chlorides in high yield. An excess of SO₂ was preferred, due to competing direct chlorination by conventional Sandmeyer chlorination. Stoichiometric iron nitrate was employed reducing co-factors that may be considered are often more expensive than iron nitrate and could interfere with the reaction chemistry. Development of a similar reaction with iron as a catalyst and an inexpensive co-factor for reduction is an exciting future challenge. CuCl can be used as a catalyst, also on larger scale. However, stoichiometric CuCl delivered higher yields for a broader range of substrates. For the chlorosulfonylation, HCl in 1,4-dioxane can be substituted with TMSCI. At 25 °C reaction temperature, reproducibility issues were encountered regarding the yield for the chlorosulfonylation to **1.** The deaminative chlorosulfonylation gives the desired sulfonyl chloride **1** in 67% yield at 25 °C. It was empirically found that increasing the temperature to 40 °C, resolved the reproducibility issues and enabled slightly higher yields for a broader range of substrates, while avoiding higher temperatures at which direct Sandmeyer chlorination becomes problematic.

The produced sulfonyl chlorides can be further converted to other sulfonic acid derivatives by subsequent addition of nucleophiles (Fig. 3). Purification of the sulfonyl chloride after deaminative chlorosulfonylation is not required. However, aqueous workup before addition of the nucleophile is recommended to achieve satisfactory yields, presumably due to interactions between nucleophile and iron or copper salts in the reaction mixture when no aqueous workup is carried out before nucleophile addition.

The iron nitrate reduction proceeds with electron rich, electron neutral, and electron deficient anilines; also amino heterocycles were converted into the respective sulfonyl chlorides. Due to the ease of purification, the products were isolated as the pyrrolidine derived sulfonamides (Fig. 4). Substrates for which the isolation or accumulation of the respective diazonium salt would be problematic, such as heterocycles **(13, 29,** and **33),** sterically hindered anilines **(24),** or complex anilines **(32,** and **34),** were functionalized using the iron nitrate reduction strategy. Anilines of which the corresponding diazonium salts are explosive, such as nitroarenes **(16),** pyridines **(13),** or benzoic acid derivatives **(15),** can be functionalized, which demonstrates a safer access to the sulfonyl chlorides compared to the functionalization of isolated or accumulated diazonium salts.

The inventors observed that electron-deficient substrates generally result in facile sulfonyl chloride formation, while complex anilines that contain electron-donating groups typically give yields <30%. The deaminative chlorosulfonylation was scaled up to 10 mmol to give **18** in 59% yield (compared to 76% yield on 0.5 mmol scale, Fig. 4).

The inventors previously demonstrated nitrate-based deaminative halogenation reactions, which remained limited to chlorination, bromination, and iodination. The discovery of the beneficial effect of iron for nitrate reduction allowed for preliminary data to now also include the remaining halide. Deaminative fluorination of anilines was achieved and demonstrated for several different aniline substrates (Fig. 5), further underlining the utility of the iron nitrate based protocol. The deaminative fluorination was carried out on sterically hindered anilines **(36)** and amino heterocycles **(39)** (Fig. 5). The inventors propose that the Fe-mediated nitrate reduction proceeds analogously to the deaminative sulfonylation reaction, producing NO₂, which can generate an aryldiazonium intermediate. The resulting diazonium salt can subsequently react with SbF₆⁻ following a conventional Balz-Schiemann mechanism.

When aqueous HCl was used instead of HCl in organic solvents, lower yields of sulfonyl chloride may be obtained. With a large excess of water present, anilines are directly converted to the respective sulfonic acids.

### Materials and Methods

All air- and moisture-insensitive reactions were carried out under ambient atmosphere and monitored by thin-layer chromatography (TLC). High-resolution mass spectra were obtained using *Q Exactive Plus* from *Thermo.* Concentration under reduced pressure was performed by rotary evaporation at 25-40°C at an appropriate pressure. Purified compounds were further dried under high vacuum (0.010-0.005 mbar). Yields refer to purified and spectroscopically pure compounds, unless otherwise stated.

### Solvents

Acetonitrile was purchased from Fisher Scientific. Anhydrous solvents were obtained from Phoenix Solvent Drying Systems. All deuterated solvents were purchased from Euriso-Top.

### Chromatography

Thin layer chromatography (TLC) was performed using EMD TLC plates pre-coated with 250 µm thickness silica gel 60 F254 plates and visualized by fluorescence quenching under 254 nm UV light or permanganate stain. Flash chromatography was performed using silica gel (40-63 µm particle size) purchased from Geduran^{®}.

### Electrochemistry

For analytical experiments, a BASI^{™} epsilon E2 device was used in combination with a BASI Cell Stand C3, purchased from Bioanalytic systems Inc. For cyclic voltammetry a MF-2013 electrode (99.95 % Pt, 1.6 mm diameter) from Bioanalytic systems Inc. was used as working electrode. Potentials were measured versus a Ag/AgCl/NaCl(aq) (3 M) reference electrode, MF-2052 electrode RE-5B form Bioanalytic systems Inc.

### EPR Spectroscopy

Room temperature CW EPR measurements were carried out at X-band (~9.45 GHz) on a Bruker Magnettech^{™} ESR5000 benchtop spectrometer equipped with a temperature-control system. A 100 kHz modulation frequency with an amplitude of 0.25 mT was used with a microwave power of ~18 mW. Each scan over the field range (187-487 mT) spectra were recorded for 180 s and were accumulated if needed. A digital RC filter with a time constant of 500 ms, implemented on ESR5000, was applied to the raw data. Both post-processing treatments were performed without artificial signal distortion. The spectra after smoothing with the Savitzky-Golay moving averages and correcting the baseline with the use of *"garlic"* function of Easyspin , MATLAB toolbox was used for further analysis.

### NMR Spectroscopy

NMR spectra were recorded on a Bruker AVANCE III HD 500 spectrometer operating at 500 MHz, 471 MHz, and 126 MHz, for ¹H, ¹⁹F, and ¹³C acquisitions, respectively; a Bruker AVANCE NEO 600 spectrometer equipped with a cryogenically cooled cryoBBO probe operating at 600 MHz and 61 MHz for ¹H and ¹⁵N acquisitions, respectively; or on a Bruker AVANCE III HD 400 spectrometer operating at 400 MHz, 100 MHz, 29 MHz, and 54 MHz for ¹H, ¹³C, ¹⁴N, and ¹⁷O acquisitions, respectively. ¹H and ¹³C Chemical shifts are reported in ppm with the solvent residual peak as the internal standard. For ¹H NMR: CDCl₃, δ 7.26; DMSO-*d₆*, δ 2.50; CD₃CN, δ 1.94; For ¹³C NMR: CDCl₃, δ 77.2; DMSO-*d₆*, δ 39.5; CD₃CN, δ 1.3. Data is reported as follows: s = singlet, d = doublet, t = triplet, q = quartet, p = pentet, m = multiplet, br = broad; coupling constants in Hz; integration. ¹⁴N and ¹⁵N shifts are reported relative to MeNO₂ (δ = 0 ppm). ¹⁷O shifts are reported relative to D₂O (δ = 0 ppm). NMR shifts of heteronuclei were referenced using their respective Ξ value using the *xiref* au program in Bruker Topspin following IUPAC recommendations.

### Computational details

All calculations were performed using a development version of the ORCA program package based on version 5.0. Geometry optimizations, transition-state locations, and enthalpy and free energy computations at standard conditions (298.15 K and 1 atm) were performed using the M06-2X-D3(0) density functional with the ma-def2-TZVP basis set, i.e., at the M06-2X-D3(0)/ma-def2-TZVP level. Electronic energies were then refined with the ma-def2-QZVP basis set. In all computations, the acetonitrile solvent effect was incorporated implicitly using the "Solvation Model based on Density (SMD)" scheme.

### Starting materials

All substrates were used as received from commercial suppliers, or prepared according to published procedures, respectively, unless otherwise stated. Anilines, nitrates, and dihaloethanes were purchased from *Sigma-Aldrich, TCl, Alfa Aesar, BLDPharm, or Chemimpex.*

### Safety statement

The procedures reported in this work are intended for use only by individuals with proper training in experimental chemistry. All hazardous materials (solid, liquid, or gaseous) should be handled using the standard work procedures described in references such as Chapter 4 of "Prudent Practices in the Laboratory". All chemical waste should be disposed of in accordance with local regulations. For general guidelines for the management of chemical waste, see Chapter 8 of "Prudent Practices in the Laboratory". Reaction set-up, and chemical-specific hazards are highlighted in bold with **"Caution:"** notes in the procedures reported in these supplementary materials. It is important to note that the absence of a caution note does not imply that no significant hazards are associated with the chemicals involved in that procedure.

### Experimental Data

### General Procedures for Deaminative Sulfonylations

### General procedure for deaminative sulfochlorination

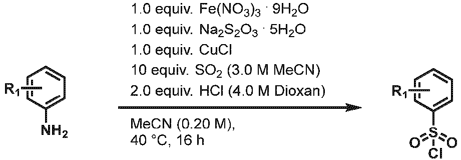

**Caution:** When performing reactions in pressurized vessels (such as closed vials, pressure tubes, and autoclaves), a blast shield must be used to minimize personal damage in case of an accident.

**0.5 mmol Scale:** Under an ambient atmosphere, to a 20-mL borosilicate vial equipped with a Teflon-coated magnetic stir bar were added the (hetero)aromatic amine (if solid, 0.500 mmol, 1.00 equiv.), Na₂S₂O₃·5H₂O (124 mg, 0.500 mmol, 1.00 equiv.) and CuCl (49.5 mg, 0.500 mmol, 1.00 equiv.). Then, acetonitrile (2.5 mL), (hetero)aromatic amine (if liquid, 0.500 mmol, 1.00 equiv.), HCl (4.0 M in 1,4-dioxane, 0.25 mL, 1.0 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(lll) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined, the solvent was removed under reduced pressure, and the residue dissolved in CDCl₃ (2.0 mL). Dibromomethane was added as internal standard (34.9 µL, 86.9 mg, 0.500 mmol, 1.00 equiv.). The mixture was filtered and a 0.5 mL aliquot of the solution used to record a ¹H-NMR spectrum. The integration of the CH₂Br₂ signal (s, 5.0 ppm) was compared to the integration of a signal corresponding to an aromatic proton of the respective sulfonyl chloride (between 6.0-8.0 ppm) to determine the yield.

### General procedure for deaminative sulfochlorination (isolation as sulfonamide)

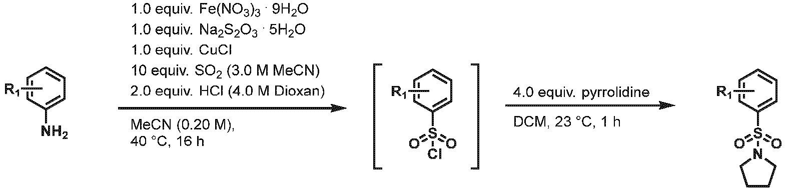

**Caution:** When performing reactions in pressurized vessels (such as closed vials, pressure tubes, and autoclaves), a blast shield must be used to minimize personal damage in case of an accident.

**0.5 mmol Scale:** Under an ambient atmosphere, to a 20-mL borosilicate vial equipped with a Teflon-coated magnetic stir bar were added the (hetero)aromatic amine (if solid, 0.500 mmol, 1.00 equiv.), Na₂S₂O₃·5H₂O (124 mg, 0.500 mmol, 1.00 equiv.) and CuCl (49.5 mg, 0.500 mmol, 1.00 equiv.). Then, acetonitrile (2.5 mL), (hetero)aromatic amine (if liquid, 0.500 mmol, 1.00 equiv.), HCl (4.0 M in 1,4-dioxane, 0.25 mL, 1.0 mmol, 2.0 equiv.), and SO₂ (3.0 M in MeCN, 1.7 mL, 5.0 mmol, 10.0 equiv.) were added, the vial was quickly sealed with a septum cap and the mixture stirred for 5 min at 23 °C. Subsequently, iron(lll) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with EtOAc (3 x 5 mL). The combined organic layers were dried over MgSO₄, filtered, and the solvent evaporated under reduced pressure. To obtain analytically pure samples of the sulfonamides, the residue was purified by chromatography on silica gel. **10 mmol Scale:** Under an ambient atmosphere, to a 350-mL Ace pressure tube equipped with a Teflon-coated magnetic stir bar were added the (hetero)aromatic amine (if solid, 10.0 mmol, 1.00 equiv.), Na₂S₂O₃·5H₂O (2.48 g, 10.0 mmol, 1.00 equiv.) and CuCl (990 mg, 10.0 mmol, 1.00 equiv.). Then, acetonitrile (50 mL), (hetero)aromatic amine (if liquid, 10.0 mmol, 1.00 equiv.), HCl (4.0 M in 1,4-dioxane, 5.0 mL, 20 mmol, 2.0 equiv.), and SO₂ (3.0 M in MeCN, 32 mL, 0.10 mol, 10 equiv.) were added, the vessel was quickly sealed and the mixture stirred for 5 min at 23 °C. Subsequently, iron(III) nitrate nonahydrate (4.04 g, 10.0 mmol, 1.00 equiv.) was added to the suspension and the pressure tube was quickly resealed. A blast shield was placed in front of the set up and the mixture was then stirred at 40 °C for 18 h. After cooling to 23 °C, the resulting mixture was transferred to a 100 mL round-bottomed flask and concentrated by rotary evaporation under reduced pressure. The residue was diluted with water (50 mL) and extracted with EtOAc (3 × 75 mL). The organic layers were concentrated in a 250 mL round-bottomed flask by rotary evaporation under reduced pressure. DCM (50 mL) was and a Teflon-coated magnetic stir bar were added, followed by pyrrolidine (3.31 mL, 2.84 g, 40.0 mmol, 4.00 equiv.). The pressure tube was sealed and the mixture stirred for 1 h at 23 °C. Subsequently, the mixture was transferred to a 250 mL separatory funnel and water (50 mL) was added, the aqueous phase decanted and further extracted with DCM (3 x 50 mL). The combined organic layers were dried over MgSO₄, filtered, and the solvent evaporated by rotary evaporation under reduced pressure. To obtain analytically pure samples of the sulfonamides, the residue was purified by chromatography on silica gel.

### General procedure for deaminative fluorination

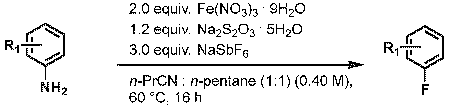

**0.5 mmol Scale:** Under an ambient atmosphere, to a 4-mL borosilicate vial equipped with a teflon-coated magnetic stir bar were added the (hetero) aromatic amine (if solid, 0.500 mmol, 1.00 equiv.), Na₂S₂O₃·5H₂O (149 mg, 0.600 mmol, 1.20 equiv.), NaSbF₆ (388 mg, 1.50 mmol, 3.00 equiv.) and iron (III) nitrate nonahydrate (404 mg, 1.00 mmol, 2.00 equiv.). Then, butyronitrile (n-PrCN) (0.625 mL), n-pentane (0.625 mL) (0.4 M overall conc.) and (hetero) aromatic amine (if liquid, 0.500 mmol, 1.00 equiv.) were added, the vial was quickly sealed with a septum cap and the reaction mixture stirred at 60 °C for 16 h. After cooling to 23 °C, the resulting mixture was passed through a pad of celite (~2.5 g) by eluting with ethyl acetate (50 ml). Then, the solvent was evaporated by rotary evaporation under reduced pressure. In order to obtain analytically pure samples of fluoroarenes, the residue was purified by chromatography on silica gel.

### General procedures for deaminative cyanation

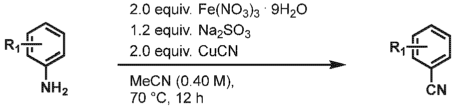

**0.5 mmol Scale:** Under an ambient atmosphere, to a 4-mL borosilicate vial equipped with a Teflon-coated magnetic stir bar were added the (hetero) aromatic amine (if solid, 0.500 mmol, 1.00 equiv.), Na₂SO₃ (75.7 mg, 0.600 mmol, 1.20 equiv.), CuCN (89.6 mg, 1.00 mmol, 2.00 equiv.) and iron (III) nitrate nonahydrate (404 mg, 1.00 mmol, 2.00 equiv.) (Fig. S1A). Then, acetonitrile (MeCN) (1.25 mL, c = 0.40 M), (hetero) aromatic amine (if liquid, 0.500 mmol, 1.00 equiv.) were added. The vessel was sealed with a septum cap (Fig. S1B) and heated at 70°C for 12 h in an aluminum block on a heating plate (Fig. S1C). After cooling to 23 °C, the resulting mixture was passed through a pad of celite by eluting with ethyl acetate (50 mL). Then, the solvent was evaporated by rotary evaporation under reduced pressure. In order to obtain analytically pure samples of benzonitriles, the residue was purified by chromatography on silica gel.

Reaction set-up for 0.500 mmol scale reactions is as follows:
**A)** Weigh all the solids in a 4-mL borosilicate vial equipped with a magnetic stir bar.
**B)** Addition of acetonitrile and sealed with a septum cap.
**C)** The reaction is heated at 70°C for 12 h in an aluminum block on a heating plate.

### Deaminative Sulfonylations with Subsequent Derivatization

### Deaminative sulfochlorination for sulfonyl chloride 4

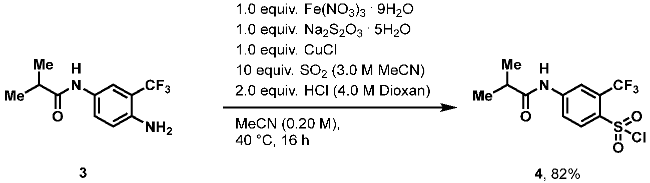

Under an ambient atmosphere, to a 20-mL borosilicate vial equipped with a Teflon-coated magnetic stir bar were added reduced Flutamide 3 (123 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with EtOAc (3 × 5 mL). The organic layers were combined in a 20-mL borosilicate vial and concentrated by rotary evaporation under reduced pressure. The residue was purified by column chromatography on silica with ethyl acetate in pentane (gradient, 0%-50% over 50 column volumes) as eluent, to give the desired sulfonyl chloride **4** as a yellow solid (0.13 g, 0.41 mmol, 82%).

**R*f*** = 0.61 (EtOAc in cyclohexane = 50%, v/v (UV)).

**HRMS-ESI (m/z)** calc'd for C₁₁H₁₀N₁O₃S₁F₃Cl₁ [M-H]⁻, 328.0028; found, 328.0029; deviation: -0.6 ppm.

### Deaminative sulfoamidation for sulfonamide 5

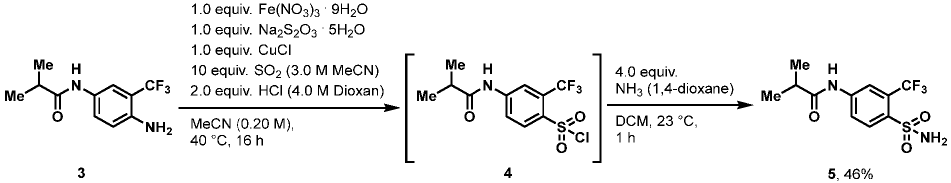

Under an ambient atmosphere, to a 20-mL borosilicate vial equipped with a Teflon-coated magnetic stir bar were added reduced Flutamide 3 (123 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with EtOAc (3 × 5 mL). The organic layers were combined in a 20-mL borosilicate vial and concentrated by rotary evaporation under reduced pressure. Then, DCM (5 mL) was added, followed by a Teflon-coated magnetic stir bar and NH₃ (0.5 M in 1,4-dioxane, 4.00 mL, 171 mg, 2.00 mmol, 4.00 equiv.). The vessel was sealed with a septum cap and the mixture stirred for 1 h at 23 °C. Subsequently, HCl solution (10% aqueous, 5 mL) was added, and the mixture extracted with EtOAc (3 × 5 mL). The combined organic layers were dried over MgSO₄, filtered, and the solvent evaporated under reduced pressure. The residue was purified by column chromatography on silica with ethyl acetate in pentane (gradient, 0%-100% over 50 column volumes) as eluent, to give the desired primary sulfonamide **5** as a white solid (70 mg, 0.23 mmol, 46%).

**R*f*** = 0.45 (MeOH in DCM = 5%, v/v (UV)).

**HRMS-EI (m/z)** calc'd for C₁₁H₁₃N₂O₃S₁F₃ [M]⁺, 310.0594; found, 310.0594; deviation: -0.3 ppm.

### Deaminative sulfoamidation for sulfonamide 6

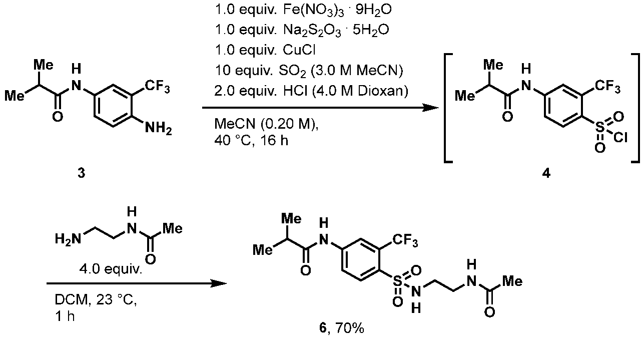

Under an ambient atmosphere, to a 20-mL borosilicate vial equipped with a Teflon-coated magnetic stir bar were added reduced Flutamide 3 (123 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with EtOAc (3 × 5 mL). The organic layers were combined in a 20-mL borosilicate vial and concentrated by rotary evaporation under reduced pressure. Then, DCM (5 mL) was added, followed by a Teflon-coated magnetic stir bar and N-acetylethylenediamine (0.213 mL, 227 mg, 2.00 mmol, 4.00 equiv.). The vessel was sealed with a septum cap and the mixture stirred for 1 h at 23 °C. Subsequently, water (5 mL) was added, and the mixture extracted with EtOAc (3 × 5 mL). The combined organic layers were dried over MgSO₄, filtered, and the solvent evaporated under reduced pressure. The residue was purified by column chromatography on silica with ethyl acetate in pentane (gradient, 0%-100% over 30 column volumes, followed by 100% over 20 column volumes) as eluent, to give the desired secondary sulfonamide **6** as a white solid (139 mg, 0.35 mmol, 70%).

**R*f*** = 0.53 (MeOH in DCM = 10%, v/v (UV)).

**HRMS-ESI (m/z)** calc'd for C₁₅H₂₀N₃O₄S₁F₃Na₁ [M+Na]⁺, 418.1019; found, 418.1015; deviation: +0.8 ppm.

### Deaminative sulfoamidation for sulfonamide 7

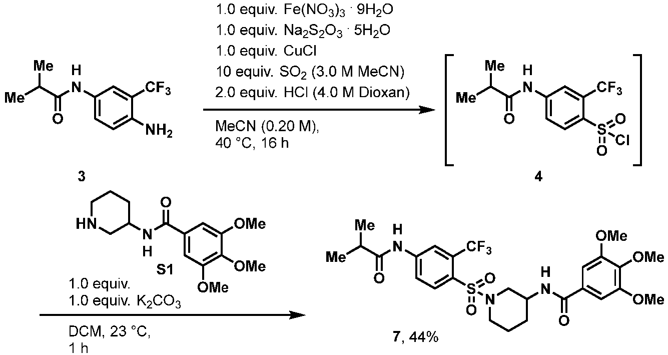

Under an ambient atmosphere, to a 20-mL borosilicate vial equipped with a Teflon-coated magnetic stir bar were added reduced Flutamide **3** (123 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with EtOAc (3 × 5 mL). The organic layers were combined in a 20-mL borosilicate vial and concentrated by rotary evaporation under reduced pressure. Then, DCM (5 mL) was added, followed by a Teflon-coated magnetic stir bar, K₂CO₃ (69.1 mg, 0.50 mmol, 1.00 equiv), and Troxipid **S1** (147 mg, 0.50 mmol, 1.00 equiv.). The vessel was sealed with a septum cap and the mixture stirred for 1 h at 23 °C. Subsequently, water (5 mL) was added, and the mixture extracted with EtOAc (3 × 5 mL). The combined organic layers were dried over MgSO₄, filtered, and the solvent evaporated under reduced pressure. The residue was purified by column chromatography on silica with ethyl acetate in pentane (gradient, 0%-100% over 50 column volumes) as eluent, to give the desired tertiary sulfonamide **7** as a white solid (129 mg, 0.22 mmol, 44%).

**R*f*** = 0.55 (MeOH in DCM = 10%, v/v (UV)).

**HRMS-ESI (m/z)** calc'd for C₂₆H₃₂N₃O₇S₁F₃Na₁ [M+Na]⁺, 610.1805; found, 610.1808; deviation: -0.5 ppm.

### Deaminative sulfofluorination for sulfonyl fluoride 8

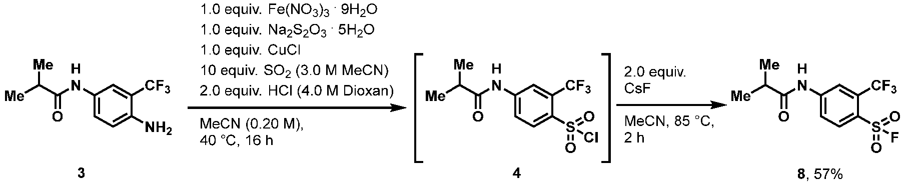

Under an ambient atmosphere, to a 20-mL borosilicate vial equipped with a Teflon-coated magnetic stir bar were added reduced Flutamide **3** (123 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with EtOAc (3 × 5 mL). The organic layers were combined in a 20-mL borosilicate vial and concentrated by rotary evaporation under reduced pressure. Then, MeCN (5 mL) was added, followed by a Teflon-coated magnetic stir bar and CsF (152 mg, 1.00 mmol, 2.00 equiv.). The vessel was sealed with a septum cap and the mixture stirred for 2 h at 85 °C. Subsequently, sat. aq. NaHCO₃ solution (5 mL) was added, and the mixture extracted with EtOAc (3 × 5 mL). The combined organic layers were dried over MgSO₄, filtered, and the solvent evaporated under reduced pressure. The residue was purified by column chromatography on silica with ethyl acetate in pentane (gradient, 0%-30% over 50 column volumes) as eluent, to give the desired sulfonyl fluoride **8** as a faint yellow solid (89 mg, 0.28 mmol, 57%).

**R*f*** = 0.60 (EtOAc in cyclohexane = 50%, v/v (UV)).

**HRMS-EI (m/z)** calc'd for C₁₁H₁₁N₁O₃S₁F₄ [M]⁺, 313.0390; found, 313.0390; deviation: +0.1 ppm.

### Deaminative sulfonic acid synthesis for sulfonic acid 9

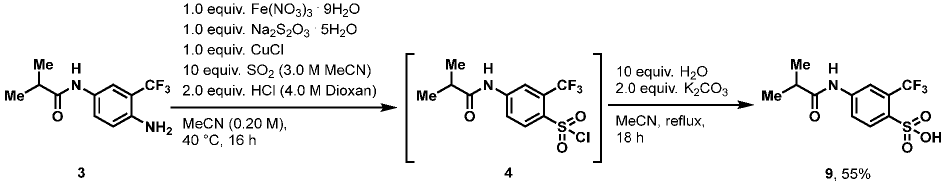

Under an ambient atmosphere, to a 20-mL borosilicate vial equipped with a Teflon-coated magnetic stir bar were added reduced Flutamide **3** (123 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with EtOAc (3 × 5 mL). The organic layers were combined in a 20-mL borosilicate vial and concentrated by rotary evaporation under reduced pressure. Then, MeCN (5 mL) was added, followed by a Teflon-coated magnetic stir bar, K₂CO₃ (138 mg, 1.00 mmol, 2.00 equiv.), and deionized H₂O (0.90 mL, 90.1 mg, 5.00 mmol, 10.0 equiv.). The vessel was sealed with a septum cap and the mixture stirred for 18 h at 85 °C. Subsequently, the solvent was evaporated and the residue purified by reversed phase column chromatography on C18 with MeOH in H₂O + 0.1% formic acid (gradient, 0%-100% over 50 column volumes) as eluent, to give the desired sulfonic acid **9** as a white solid (86 mg, 0.28 mmol, 55%).

**R*f*** = 0.29 (MeOH in DCM = 20%, v/v (UV)).

**HRMS-EI (m/z)** calc'd for C₁₁H₁₁N₁O₄S₁F₃ [M]⁺, 310.0366; found, 310.0369; deviation: -0.9 ppm.

### Aniline Scope for Deaminative Sulfochlorination (isolation as sulfonamides) 4-(Pyrrolidin-1-ylsulfonyl)benzonitrile (10)

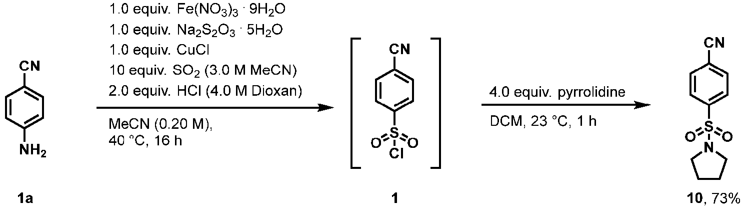

Under an ambient atmosphere, to a 20-mL borosilicate vial equipped with a Teflon-coated magnetic stir bar were added 4-aminobenzonitrile (59.1 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (gradient, 0%-50% over 30 column volumes) as eluent, to give the desired sulfonamide 10 as a colorless solid (86 mg, 0.36 mmol, 73%).

**R*f*** = 0.21 (EtOAc in cyclohexane = 20%, v/v (UV)).

**HRMS-EI (m/z)** calc'd for C₁₁H₁₂N₂O₂S₁ [M]⁺, 236.0614; found, 236.0614; deviation: +0.1 ppm.

### 1-((4-Phenoxyphenyl)sulfonyl)pyrrolidine (11)

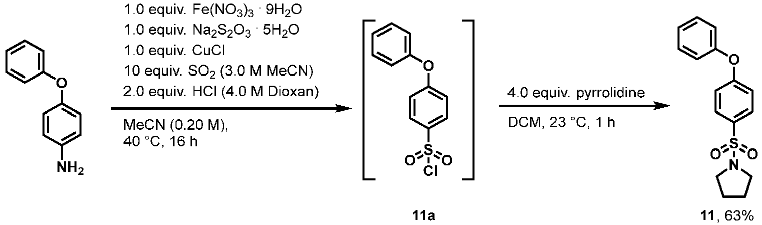

Under an ambient atmosphere, to a 20-mL borosilicate vial equipped with a Teflon-coated magnetic stir bar were added 4-phenoxyaniline (92.3 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (gradient, 0%-50% over 30 column volumes) as eluent, to give the desired sulfonamide **11** as a colorless solid (95 mg, 0.31 mmol, 63%).

**R*f*** = 0.42 (EtOAc in cylohexane = 20%, v/v (UV)).

**HRMS-EI (m/z)** calc'd for C₁₆H₁₇N₁O₃S₁ [M]⁺, 303.0924; found, 303.0928; deviation: -1.3 ppm.

### 1-((4-Methoxyphenyl)sulfonyl)pyrrolidine (12)

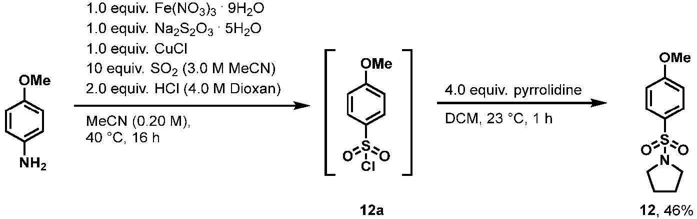

Under an ambient atmosphere, to a 4-mL borosilicate vial equipped with a Teflon-coated magnetic stir bar were added 4-methoxyaniline (12.3 mg, 0.100 mmol, 1.00 equiv.), Na₂S₂O₃·5H₂O (24.8 mg, 0.100 mmol, 1.00 equiv.) and CuCl (9.90 mg, 0.100 mmol, 1.0 equiv.). Then, acetonitrile (0.5 mL), HCl (4.0 M in 1,4-dioxane, 50 µL, 0.10 mmol, 2.0 equiv.), and SO₂ (3.0M in MeCN, 0.323 mL, 1.00 mmol, 10.0 equiv.) were added, the vial was quickly sealed with a septum cap and the mixture stirred for 5 min at 23 °C. Subsequently, iron(III) nitrate nonahydrate (40.4 mg, 0.100 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (2 mL) and extracted with DCM (3 × 2 mL). The organic layers were combined in a 4-mL borosilicate vial and a Teflon-coated magnetic stir bar was added, followed by pyrrolidine (33.1 µL, 28.4 mg, 0.400 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (gradient, 0%-50% over 30 column volumes) as eluent, to give the desired sulfonamide **12** as a colorless solid (11.2 mg, 0.46 mmol, 46%).

**R*f*** = 0.22 (EtOAc in pentane = 20%, v/v (UV)).

**HRMS-EI (m/z)** calc'd for C₁₁H₁₅N₁O₃S₁ [M]⁺, 241.0767; found, 241.0765; deviation: -0.77 ppm.

### 2-Chloro-3-(pyrrolidin-1-ylsulfonyl)pyridine (13)

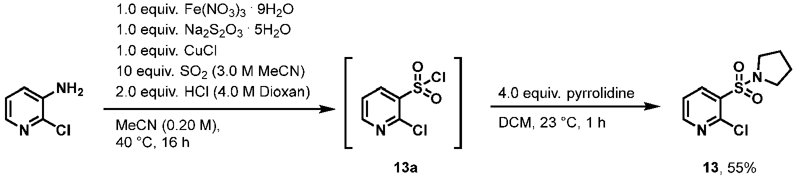

Under an ambient atmosphere, to a 20-mL borosilicate vial equipped with a Teflon-coated magnetic stir bar were added 3-amino-2-chloropyridine (64.3 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (15%, v/v) as eluent, to give the desired sulfonamide **13** as a colorless oil (68 mg, 0.28 mmol, 55%).

**R*f*** = 0.25 (EtOAc in pentane = 40%, v/v (UV)).

**HRMS-ESI (m/z)** calc'd for C₉H₁₁N₂O₂S₁Cl₁Na₁ [M+Na]⁺, 269.0122; found, 269.0122; deviation: +0.2 ppm.

### 1-((4'-Chloro-[1,1'-biphenyl]-2-yl)sulfonyl)pyrrolidine (14)

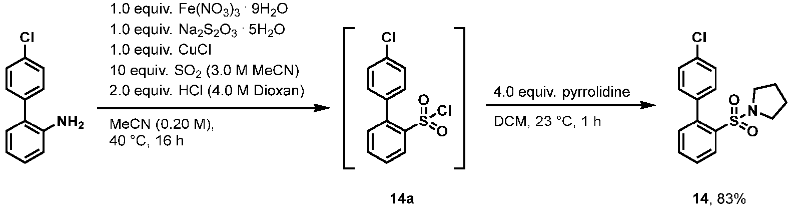

Under an ambient atmosphere, to a 20-mL borosilicate vial equipped with a Teflon-coated magnetic stir bar were added 4-chloro-(1-biphenyl)-2-amine (102 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (gradient, 0%-50% over 30 column volumes) as eluent, to give the desired sulfonamide **14** as a colorless solid (0.13 mg, 0.42 mmol, 83%).

**R*f*** = 0.49 (EtOAc in cyclohexane = 20%, v/v (UV)).

**HRMS-EI (m/z)** calc'd for C₁₆H₁₆N₂O₂S₁Cl₁ [M]⁺, 321.0585; found, 321.0588; deviation: -1.2 ppm.

### 3-(Pyrrolidin-1-ylsulfonyl)-5-(trifluoromethyl)benzoic acid (15)

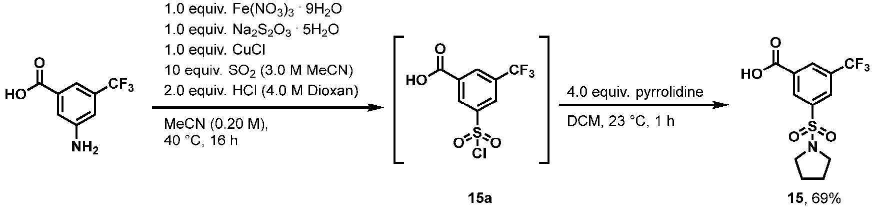

Under an ambient atmosphere, to a 20-mL borosilicate vial equipped with a Teflon-coated magnetic stir bar were added 3-amino-5-(trifluormethyl)-benzoic acid (103 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (38% + 2% AcOH) as eluent, to give the desired sulfonamide **15** as an off-white solid (0.11 mg, 0.35 mmol, 69%).

**R*f*** = 0.74 (EtOAc in pentane = 28% + 2% AcOH, v/v (UV)).

**HRMS-ESI (m/z)** calc'd for C₁₂H₁₂N₁O₄S₁F₃Na₁ [M+Na]⁺, 346.0331; found, 346.0332; deviation: -0.1 ppm.

### 4-(Pyrrolidin-1-ylsulfonyl)nitrobenzene (16)

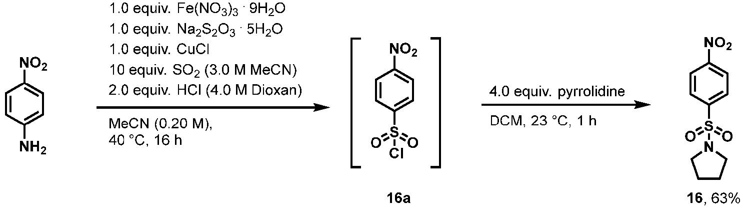

Under an ambient atmosphere, to a 20-mL borosilicate vial equipped with a Teflon-coated magnetic stir bar were added 4-nitroaniline (69.1 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (12%, v/v) as eluent, to give the desired sulfonamide **16** as a yellow solid (81 mg, 0.32 mmol, 63%).

**R*f*** = 0.37 (EtOAc in pentane = 12%, v/v (UV)).

**HRMS-EI (m/z)** calc'd for C₁₀H₁₂N₂O₄S₁ [M]⁺, 256.0512; found, 256.0512; deviation: +0.3 ppm.

### 1-((2-Chloro-4-iodophenyl)sulfonyl)pyrrolidine (17)

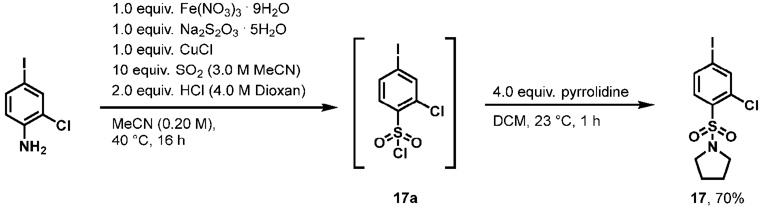

Under an ambient atmosphere, to a 20-mL borosilicate vial equipped with a Teflon-coated magnetic stir bar were added 2-chloro-4-iodaniline (127 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (gradient, 0%-50% over 30 column volumes) as eluent, to give the desired sulfonamide **17** as a colorless solid (0.130 g, 0.35 mmol, 70%).

**R*f*** = 0.52 (EtOAc in cyclohexane = 20%, v/v (UV)).

**HRMS-EI (m/z)** calc'd for C₁₀H₁₁N₁O₂S₁Cl₁I₁ [M]⁺, 370.9238; found, 370.9242; deviation: -1.0 ppm.

### Phenyl(4-(pyrrolidin-1-ylsulfonyl)phenyl)methanone (18)

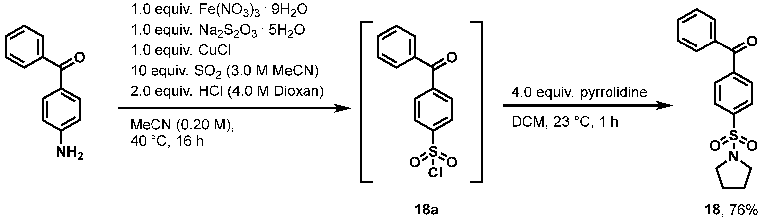

**0.5 mmol Scale:** Under an ambient atmosphere, to a 20-mL borosilicate vial equipped with a Teflon-coated magnetic stir bar were added 4-aminobenzophenone (98.6 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (gradient, 0%-50% over 30 column volumes) as eluent, to give the desired sulfonamide **18** as a colorless solid (0.12 g, 0.38 mmol, 76%).

**10 mmol Scale:** Under an ambient atmosphere, to a 350-mL Ace pressure tube equipped with a Teflon-coated magnetic stir bar were added the 4-aminobenzophenone (1.97 g, 10.0 mmol, 1.00 equiv.), Na₂S₂O₃·5H₂O (2.48 g, 10.0 mmol, 1.00 equiv.) and CuCl (990 mg, 10.0 mmol, 1.00 equiv.). Then, acetonitrile (50 mL), HCl (4.0 M in 1,4-dioxane, 5.0 mL, 20 mmol, 2.0 equiv.), and SO₂ (3.00 M in MeCN, 32.3 mL, 100 mmol, 10.0 equiv.) were added, the vessel was quickly sealed and the mixture stirred for 5 min at 23 °C. Subsequently, iron(III) nitrate nonahydrate (4.04 g, 10.0 mmol, 1.00 equiv.) was added to the suspension and the pressure tube was quickly resealed. A blast shield was placed in front of the set up and the mixture was then stirred at 40 °C for 18 h. After cooling to 23 °C, the resulting mixture was transferred to a 100 mL round-bottomed flask and concentrated by rotary evaporation under reduced pressure. The residue was diluted with water (50 mL) and extracted with EtOAc (3 × 75 mL). The organic layers were concentrated in a 250 mL round-bottomed flask by rotary evaporation under reduced pressure. DCM (50 mL) and a Teflon-coated magnetic stir bar were added, followed by pyrrolidine (3.31 mL, 2.84 g, 40.0 mmol, 4.00 equiv.). The pressure tube was sealed and the mixture stirred for 1 h at 23 °C. Subsequently, the mixture was transferred to a 250 mL separatory funnel and water (50 mL) was added, the aqueous phase decanted and further extracted with DCM (3 x 50 mL). The combined organic layers were dried over MgSO₄, filtered, and the solvent evaporated by rotary evaporation under reduced pressure. The residue was purified by column chromatography on silica with ethyl acetate in pentane (gradient, 0% for 10 column volumes, then 0%-50% over 50 column volumes) as eluent, to give the desired sulfonamide **18** as a faint red solid (1.85 g, 5.87 mmol, 59%).

**10 mmol Scale and catalytic CuCl:** Under an ambient atmosphere, to a 350-mL Ace pressure tube equipped with a Teflon-coated magnetic stir bar were added the 4-aminobenzophenone (1.97 g, 10.0 mmol, 1.00 equiv.), Na₂S₂O₃·5H₂O (2.48 g, 10.0 mmol, 1.00 equiv.) and CuCl (197 mg, 2.00 mmol, 20 mol%). Then, acetonitrile (50 mL), HCl (4.0 M in 1,4-dioxane, 5.0 mL, 20 mmol, 2.0 equiv.), and SO₂ (3.0 M in MeCN, 32 mL, 0.10 mol, 10 equiv.) were added, the vessel was quickly sealed and the mixture stirred for 5 min at 23 °C. Subsequently, iron(III) nitrate nonahydrate (4.04 g, 10.0 mmol, 1.00 equiv.) was added to the suspension and the pressure tube was quickly resealed. A blast shield was placed in front of the set up and the mixture was then stirred at 40 °C for 18 h. After cooling to 23 °C, the resulting mixture was transferred to a 100 mL round-bottomed flask and concentrated by rotary evaporation under reduced pressure. The residue was diluted with water (50 mL) and extracted with EtOAc (3 × 75 mL). The organic layers were concentrated in a 250 mL round-bottomed flask by rotary evaporation under reduced pressure. DCM (50 mL) and a Teflon-coated magnetic stir bar were added, followed by pyrrolidine (3.31 mL, 2.84 g, 40.0 mmol, 4.00 equiv.). The pressure tube was sealed and the mixture stirred for 1 h at 23 °C. Subsequently, the mixture was transferred to a 250 mL separatory funnel and water (50 mL) was added, the aqueous phase decanted and further extracted with DCM (3 x 50 mL). The combined organic layers were dried over MgSO₄, filtered, and the solvent evaporated by rotary evaporation under reduced pressure. Subsequently, CDCl₃ (5 mL) and 1,3,5-trimethoxybenzene (1.68 g, 10.0 mmol, 1.00 equiv.) as internal standard were added. A 0.5 mL aliquot was taken and the yield determined to be 68 % by ¹H NMR spectroscopy at 500 MHz and 298 K by comparing the integration of the 1,3,5-trimethoxybenzene signal at 6.06 ppm (s, 3H) to the signal of **18** at 7.62 ppm (t, 7.6 Hz, 1H).

**R*f*** = 0.28 (EtOAc in cyclohexane = 20%, v/v (UV)).

**HRMS-EI (m/z)** calc'd for C₁₇H₁₇N₁O₃S₁ [M]⁺, 315.0924; found, 315.0929; deviation: -1.6 ppm.

### 1-((6-Bromonaphthalen-2-yl)sulfonyl)pyrrolidine (19)

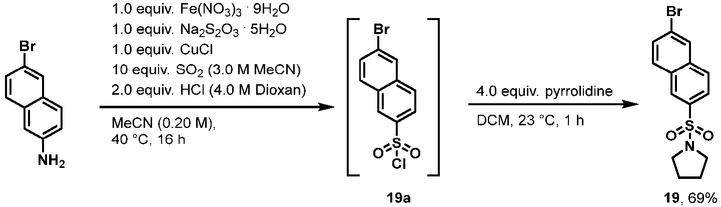

Under an ambient atmosphere, to a 20-mL borosilicate vial equipped with a Teflon-coated magnetic stir bar were added 6-bromnaphthalene-2-amine (111 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (20%, v/v) as eluent, to give the desired sulfonamide **19** as a brown solid (69 mg, 0.35 mmol, 69%).

**R*f*** = 0.33 (EtOAc in pentane = 20%, v/v (UV)).

**HRMS-EI (m/z)** calc'd for C₁₄H₁₄N₁O₂S₁Br₁ [M]⁺, 338.9923; found, 338.9925; deviation: -0.6 ppm.

### 2-Chloro-4-(pyrrolidin-1-ylsulfonyl)benzonitrile (20)

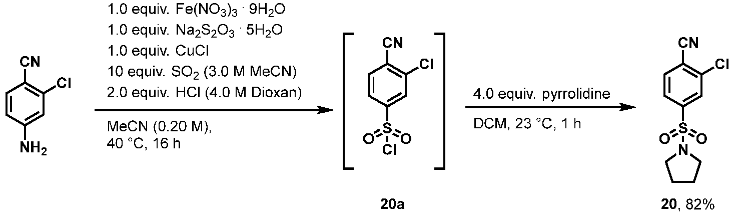

Under an ambient atmosphere, to a 20-mL borosilicate vial equipped with a Teflon-coated magnetic stir bar were added 4-amino-2-chlorobenzonitrile (76.3 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (12%, v/v) as eluent, to give the desired sulfonamide 20 as an off-white solid (87 mg, 0.41 mmol, 82%).

**R*f*** = 0.34 (EtOAc in pentane = 30%, v/v (UV)).

**HRMS-EI (m/z)** calc'd for C₁₁H₁₁N₂O₂S₁Cl₁ [M]⁺, 270.0224; found, 270.0226; deviation: -0.8 ppm.

### 4-(Pyrrolidin-1-ylsulfonyl)trifluoromethoxybenzene (21)

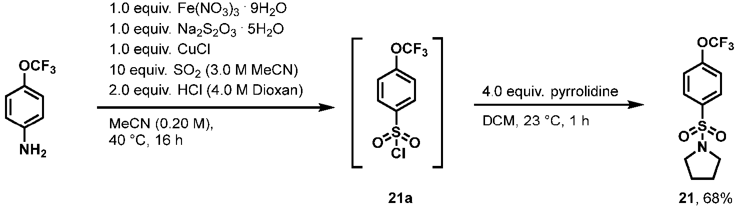

Under an ambient atmosphere, to a 20-mL borosilicate vial equipped with a Teflon-coated magnetic stir bar were added 4-trifluoromethoxyaniline (88.6 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (gradient, 0%-50% over 30 column volumes) as eluent, to give the desired sulfonamide **21** as a colorless solid (99 mg, 0.34 mmol, 68%).

**R*f*** = 0.38 (EtOAc in cyclohexane = 20%, v/v (UV)).

**HRMS-EI (m/z)** calc'd for C₁₁H₁₂N₁O₃S₁F₃ [M]⁺, 295.0485; found, 295.0489; deviation: -1.5 ppm.

### 1-((2-((Trifluoromethyl)thio)phenyl)sulfonyl)pyrrolidine (22)

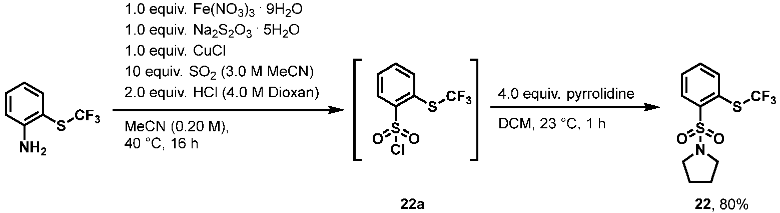

Under an ambient atmosphere, to a 20-mL borosilicate vial equipped with a Teflon-coated magnetic stir bar were added 2-(trifluoromethylthio)aniline (96.6 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (12% v/v) as eluent, to give the desired sulfonamide 22 as a yellow oil (98 mg, 0.40 mmol, 80%). **R*f*** = 0.47 (EtOAc in pentane = 20%, v/v (UV)).

**HRMS-EI (m/z)** calc'd for C₁₁H₁₂N₁O₂S₂F₃ [M]⁺, 311.0256; found, 311.0258; deviation: -0.6 ppm.

### 3-Bromo-4-(pyrrolidin-1-ylsulfonyl)benzonitrile (23)

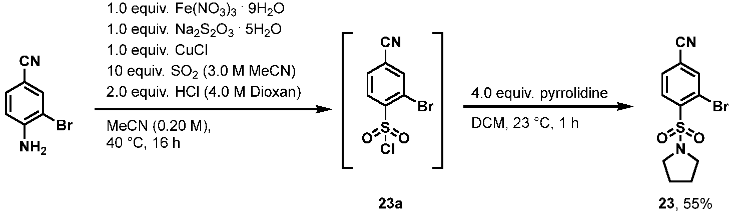

Under an ambient atmosphere, to a 20-mL borosilicate vial equipped with a Teflon-coated magnetic stir bar were added 4-amino-3-bromobenzonitrile (98.5 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (14%, v/v) as eluent, to give the desired sulfonamide **23** as an off-white solid (87 mg, 0.28 mmol, 55%).

**R*f*** = 0.40 (EtOAc in pentane = 30%, v/v (UV)).

**HRMS-ESI (m/z)** calc'd for C₁₁H₁₁N₂O₂S₁Br₁Na₁ [M+Na]⁺, 336.9617; found, 336.9615; deviation: +0.7 ppm.

### 1-((2-Methyl-3-nitrophenyl)sulfonyl)pyrrolidine (24)

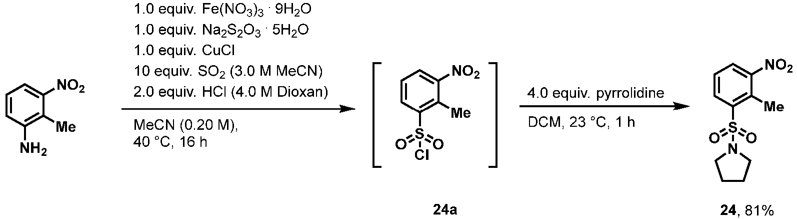

Under an ambient atmosphere, to a 20-mL borosilicate vial equipped with a Teflon-coated magnetic stir bar were added 2-methyl-3-nitroaniline (76.1 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (gradient, 0%-50% over 30 column volumes) as eluent, to give the desired sulfonamide **24** as a colorless solid (0.11 g, 0.41 mmol, 81%).

**R*f*** = 0.28 (EtOAc in pentane = 20%, v/v (UV)).

**HRMS-EI (m/z)** calc'd for C₁₁H₁₄N₂O₄S₁ [M]⁺, 270.0669; found, 270.0669; deviation: -0.4 ppm.

### 4-(pyrrolidin-1-ylsulfonyl)benzenesulfonamide (25)

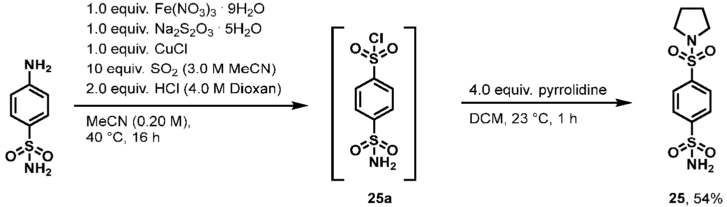

Under an ambient atmosphere, to a 20-mL borosilicate vial equipped with a Teflon-coated magnetic stir bar were added sulfanilamide (59.1 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (40%, v/v) as eluent, to give the desired sulfonamide 25 as a colorless solid (78 mg, 0.27 mmol, 54%).

**R*f*** = 0.64 (EtOAc in pentane = 70%, v/v (UV)).

**HRMS-ESI (m/z)** calc'd for C₁₀H₁₄N₂O₄S₂Na₁ [M+Na]⁺, 313.0287; found, 313.0289; deviation: -0.5 ppm.

### 1-((3,5-Dibromo-4-methylphenyl)sulfonyl)pyrrolidine (26)

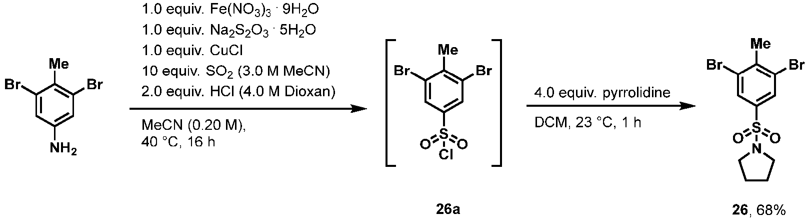

Under an ambient atmosphere, to a 20-mL borosilicate vial equipped with a Teflon-coated magnetic stir bar were added 3,5-dibromo-4-methylaniline (133 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (gradient, 0%-50% over 30 column volumes) as eluent, to give the desired sulfonamide 26 as a colorless solid (0.13 g, 0.34 mmol, 68%).

**R*f*** = 0.51 (EtOAc in pentane = 20%, v/v (UV)).

**HRMS-EI** (m/z) calc'd for C₁₁H₁₃N₁O₂S₁Br₂ [M]⁺, 380.9029; found, 380.9034; deviation: -1.4 ppm.

### 4-(Pyrrolidin-1-ylsulfonyl)difluoromethoxybenzene (27)

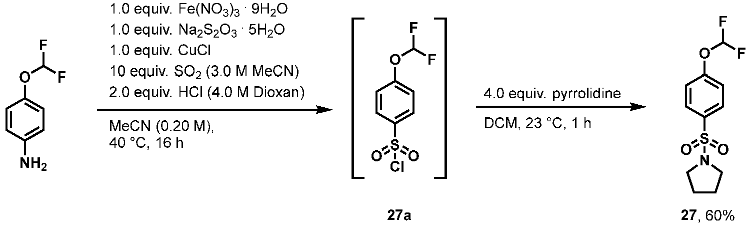

Under an ambient atmosphere, to a 20-mL borosilicate vial equipped with a Teflon-coated magnetic stir bar were added 4-difluoromethoxyaniline (79.6 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (gradient, 0%-50% over 30 column volumes) as eluent, to give the desired sulfonamide **27** as a colorless solid (82 mg, 0.30 mmol, 60%).

**R*f*** = 0.26 (EtOAc in cyclohexane = 20%, v/v (UV)).

**HRMS-EI (m/z)** calc'd for C₁₁H₁₃N₁O₃S₁F₂ [M]⁺, 277.0579; found, 277.0579; deviation: -0.1 ppm.

### 4-Methyl-7-(pyrrolidin-1-ylsulfonyl)-2H-chromen-2-one (28)

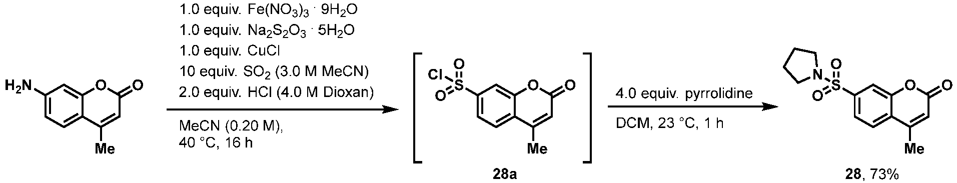

Under an ambient atmosphere, to a 20-mL borosilicate vial equipped with a Teflon-coated magnetic stir bar were added 7-amino-4-methylcumarin (87.6 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in cyclohexane (gradient, 0%-100% over 30 column volumes) as eluent, to give the desired sulfonamide **28** as a colorless solid (107 mg, 0.37 mmol, 73%).

**R*f*** = 0.27 (EtOAc in cyclohexane = 50%, v/v (UV)).

**HRMS-EI (m/z)** calc'd for C₁₄H₁₅N₁O₄S₁ [M]⁺, 293.0716; found, 293.0714; deviation: +0.9 ppm.

### Methyl 3-(pyrrolidin-1-ylsulfonyl)thiophene-2-carboxylate (29)

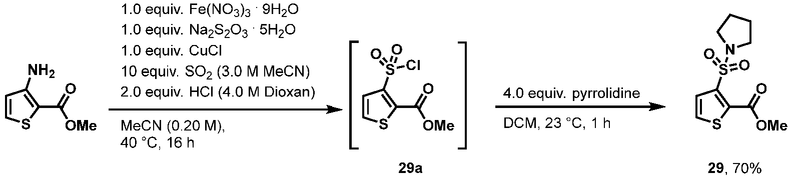

Under an ambient atmosphere, to a 20-mL borosilicate vial equipped with a Teflon-coated magnetic stir bar were added methyl 3-amino-2-thiophenecarboxylate (78.6 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (13%, v/v) as eluent, to give the desired sulfonamide **29** as a colorless oil (96 mg, 0.35 mmol, 70%).

**R*f*** = 0.34 (EtOAc in pentane = 40%, v/v (UV)).

**HRMS-ESI (m/z)** calc'd for C₁₀H₁₃N₁O₄S₂Na₁ [M+Na]⁺, 298.0178; found, 298.0175; deviation: +1.1 ppm.

### 1-((2,2-Difluorobenzo[d][1,3]dioxol-4-yl)sulfonyl)pyrrolidine (30)

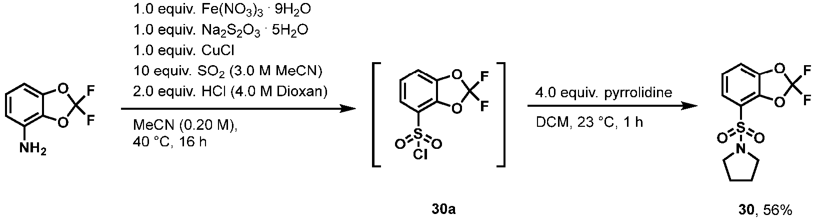

Under an ambient atmosphere, to a 20-mL borosilicate vial equipped with a Teflon-coated magnetic stir bar were added 2,2-difluorobenzo[d][1,3]dioxol-5-amine (86.6 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (gradient, 0%-50% over 30 column volumes) as eluent, to give the desired sulfonamide 30 as a colorless solid (81 mg, 0.28 mmol, 56%).

**R*f*** = 0.36 (EtOAc in pentane = 20%, v/v (UV)).

**HRMS-EI (m/z)** calc'd for C₁₁H₁₁N₁O₄S₁F₂ [M]⁺, 291.0371; found, 291.0372; deviation: -0.1 ppm.

### Hippuric acid-derived sulfonamide 31

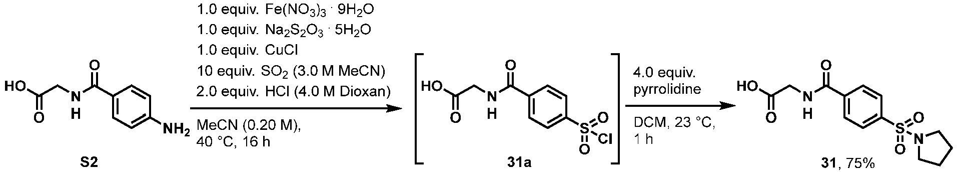

Under an ambient atmosphere, to a 20-mL borosilicate vial equipped with a Teflon-coated magnetic stir bar were added reduced p-aminohippuric acid **S2** (97.1 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with MeOH in DCM (10% + 2% AcOH, v/v) as eluent, to give the desired sulfonamide **33** as a light-yellow solid (0.12 g, 0.38 mmol, 75%).

**R*f*** = 0.61 (MeOH in DCM = 10% + 2% AcOH, v/v (UV)).

**HRMS-ESI (m/z)** calc'd for C₁₃H₁₅N₂O₅S₁ [M-H]⁻, 311.0707; found, 311.0710; deviation: -0.7 ppm.

### Flutamide-derived sulfonamide 32

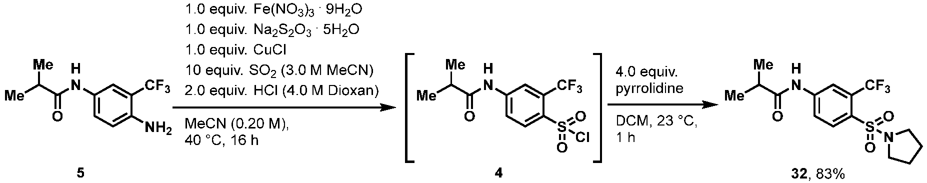

Under an ambient atmosphere, to a 20-mL borosilicate vial equipped with a Teflon-coated magnetic stir bar were added reduced Flutamide 5 (123 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (25%, v/v) as eluent, to give the desired sulfonamide **32** as a light-yellow oil (0.12 g, 0.42 mmol, 83%).

**R*f*** = 0.28 (EtOAc in pentane = 40%, v/v (UV)).

**HRMS-ESI (m/z)** calc'd for C₁₅H₁₉N₂O₃S₁F₃Na₁ [M+Na]⁺, 387.0961; found, 387.0959; deviation: +0.3 ppm.

### 5-(Pyrrolidin-1-ylsulfonyl)picolinonitrile (33)

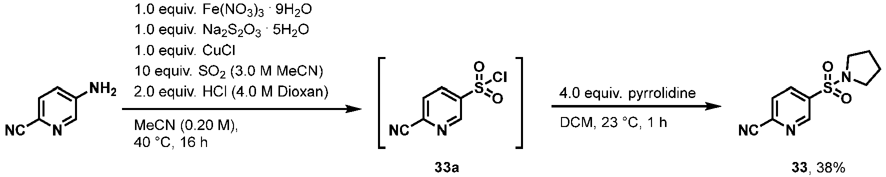

Under an ambient atmosphere, to a 20-mL borosilicate vial equipped with a Teflon-coated magnetic stir bar were added 5-aminopicolinonitrile (59.6 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The organic layers were combined in a 20-mL borosilicate vial and a Teflon-coated magnetic stir bar was added, followed by pyrrolidine (165 µL, 142 mg, 2.00 mmol, 4.00 equiv.). The vessel was sealed with a septum cap and the mixture stirred for 1 h at 23 °C. Subsequently water (5 mL) was added, the aqueous phase decanted and further extracted with DCM (3 × 5 mL). The combined organic layers were dried over MgSO₄, filtered, and the solvent evaporated under reduced pressure. The residue was purified by column chromatography on silica with ethyl acetate in pentane (25%, v/v) as eluent, to give the desired sulfonamide **35** as an off-white solid (45 mg, 0.19 mmol, 38%).

**R*f*** = 0.50 (EtOAc in pentane = 40%, v/v (UV)).

**HRMS-EI (m/z)** calc'd for C₁₀H₁₁N₃O₂S₁ [M]⁺, 237.0567; found, 237.0565; deviation: +0.6 ppm.

### Sulfadoxin-derived sulfonyl chloride 34a

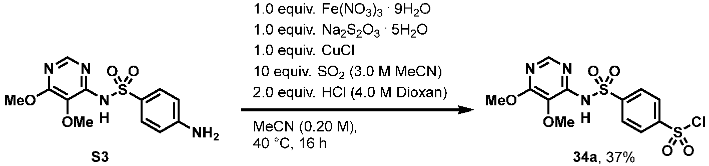

Under an ambient atmosphere, to a 20-mL borosilicate vial equipped with a Teflon-coated magnetic stir bar were added Sulfadoxine **S3** (155.2 mg, 0.500 mmol, 1.00 equiv.), Na₂S₂O₃·5H₂O (124 mg, 0.500 mmol, 1.00 equiv.) and CuCl (49.5 mg, 0.500 mmol, 1.0 equiv.). Then, acetonitrile (2.5 mL), HCl (4.0 M in 1,4-dioxane, 250 µL, 1.00 mmol, 2.0 equiv.), and SO₂ (3.0 M in MeCN, 1.7 mL, 5.0 mmol, 10 equiv.) were added, the vial was quickly sealed with a septum cap and the mixture stirred for 5 min at 23 °C. Subsequently, iron(III) nitrate nonahydrate (202 mg, 0.500 mmol, 1.00 equiv.) was added to the suspension and the vial was quickly resealed with a septum cap. The mixture was then stirred at 40 °C for 18 h. After cooling to 23 °C, the resulting mixture was concentrated by rotary evaporation under reduced pressure. The residue was diluted with water (5 mL) and extracted with DCM (3 × 5 mL). The combined organic layers were dried over MgSO₄, filtered, and the solvent evaporated under reduced pressure. The residue was purified by column chromatography on silica with ethyl acetate in pentane (30%, v/v) as eluent, to give the desired sulfonyl chloride **34** as a colorless solid (73 mg, 0.19 mmol, 37%).

**R*f*** = 0.25 (EtOAc in pentane = 50%, v/v (UV)).

**HRMS-ESI (m/z)** calc'd for C₁₂H₁₁N₃O₆S₂Cl₁ [M-H]⁻, 391.9783; found, 391.9789; deviation: +1.4 ppm.

### Aniline Scope for Deaminative Fluorination

### Ethyl-4-fluorobenzoate (35)

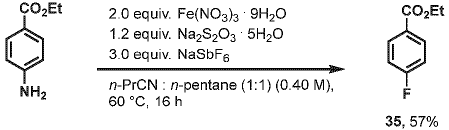

Under an ambient atmosphere, to a 4-mL borosilicate vial equipped with a teflon-coated magnetic stir bar were added ethyl-4-aminobenzoate (16.5 mg, 0.100 mmol, 1.00 equiv.), Na₂S₂O₃·5H₂O (29.8 mg, 0.120 mmol, 1.20 equiv.), NaSbF₆ (77.6 mg, 0.300 mmol, 3.00 equiv.) and iron (III) nitrate nonahydrate (80.8 mg, 0.200 mmol, 2.00 equiv.). Then, butyronitrile (n-PrCN) (0.125 mL), n-pentane (0.125 mL) (0.4 M overall conc.) were added, the vial was quickly sealed with a septum cap and the reaction mixture stirred at 60 °C for 16 h. After cooling to 23 °C, the resulting mixture was passed through a pad of celite (~1.0 g) by eluting with ethyl acetate (10 ml). Then, the solvent was removed under reduced pressure. The residue was purified by column chromatography on silica with ethyl acetate in pentane (1%, v/v) as eluent, to give the desired fluoroarene 35 as a colorless liquid (9.6 mg, 0.057 mmol, 57%).

**R*f*** = 0.53 (EtOAc in pentane = 5%, v/v (UV)).

**HRMS-EI (m/z)** calc'd for C₉H₉O₂F₁ [M]⁺, 168.0581; found, 168.0581; deviation: -0.2 ppm.

### 4-Fluoro-3,5-dimethylbenzonitrile (36)

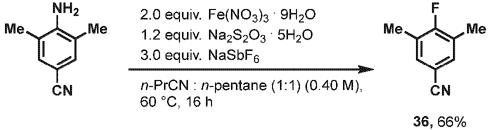

Under an ambient atmosphere, to a 4-mL borosilicate vial equipped with a teflon-coated magnetic stir bar were added 4-amino-3,5-dimethylbenzonitrile (73.1 mg, 0.500 mmol, 1.00 equiv.), Na₂S₂O₃·5H₂O (149 mg, 0.600 mmol, 1.20 equiv.), NaSbF₆ (388 mg, 1.50 mmol, 3.00 equiv.) and iron (III) nitrate nonahydrate (404 mg, 1.00 mmol, 2.00 equiv.). Then, butyronitrile (n-PrCN) (0.625 mL), n-pentane (0.625 mL) (0.4 M overall conc.) were added, the vial was quickly sealed with a septum cap and the reaction mixture stirred at 60 °C for 16 h. After cooling to 23 °C, the resulting mixture was passed through a pad of celite (~2.5 g) by eluting with ethyl acetate (50 ml). Then, the solvent was removed under reduced pressure. The residue was purified by column chromatography on silica with ethyl acetate in pentane (1%, v/v) as eluent, to give the desired fluoroarene 36 as a colorless solid (49 mg, 0.33 mmol, 66%).

**R*f*** = 0.43 (EtOAc in pentane = 5%, v/v (UV)).

**HRMS-EI (m/z)** calc'd for C₉H₈N₁F₁ [M]⁺, 149.0635; found, 149.0636; deviation: -0.2 ppm.

### (4-Fluorophenyl)(phenyl)methanone (37)

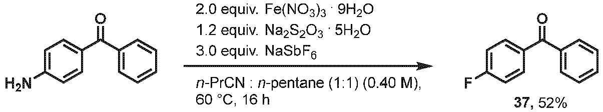

Under an ambient atmosphere, to a 4-mL borosilicate vial equipped with a teflon-coated magnetic stir bar were added 4-aminobenzophenone (19.7 mg, 0.100 mmol, 1.00 equiv.), Na₂S₂O₃·5H₂O (29.8 mg, 0.120 mmol, 1.20 equiv.), NaSbF₆ (77.6 mg, 0.300 mmol, 3.00 equiv.) and iron (III) nitrate nonahydrate (80.8 mg, 0.200 mmol, 2.00 equiv.). Then, butyronitrile (n-PrCN) (0.125 mL), n-pentane (0.125 mL) (0.4 M overall conc.) were added, the vial was quickly sealed with a septum cap and the reaction mixture stirred at 60 °C for 16 h. After cooling to 23 °C, the resulting mixture was passed through a pad of celite (~1.0 g) by eluting with ethyl acetate (10 ml). Then, the solvent was removed under reduced pressure. The residue was purified by column chromatography on silica with ethyl acetate in pentane (1%, v/v) as eluent, to give the desired fluoroarene **37** as a colorless liquid (10.2 mg, 0.052 mmol, 52%).

**R*f*** = 0.35 (EtOAc in pentane = 5%, v/v (UV)).

### NMR Spectroscopy:

**HRMS-EI (m/z)** calc'd for C₁₃H₉O₁F₁ [M]⁺, 200.0632; found, 200.0635; deviation: -1.4 ppm.

### 1,1,1,3,3,3-Hexafluoro-2-(4-fluorophenyl)propan-2-ol (38)

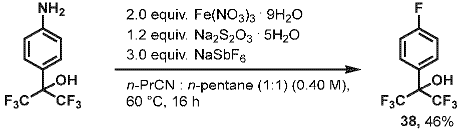

Under an ambient atmosphere, to a 4-mL borosilicate vial equipped with a teflon-coated magnetic stir bar were added 2-(4-aminophenyl)-1,1,1,3,3,3-hexafluor-2-propanol (130 mg, 0.500 mmol, 1.00 equiv.), Na₂S₂O₃·5H₂O (149 mg, 0.600 mmol, 1.20 equiv.), NaSbF₆ (388 mg, 1.50 mmol, 3.00 equiv.) and iron (III) nitrate nonahydrate (404 mg, 1.00 mmol, 2.00 equiv.). Then, butyronitrile (*n*-PrCN) (0.625 mL), n-pentane (0.625 mL) (0.4 M overall conc.) were added, the vial was quickly sealed with a septum cap and the reaction mixture stirred at 60 °C for 16 h. After cooling to 23 °C, the resulting mixture was passed through a pad of celite (~2.5 g) by eluting with CH₂Cl₂ (50 ml). Then, the solvent was removed under reduced pressure. The yield was determined by both ¹H NMR spectroscopy at 500 MHz dissolving the residue of the reaction mixture in 0.5 mL of CDCl₃, and using CH₂Br₂ as internal standard (34.9 µL, 86.9 mg, 0.500 mmol, 1.00 equiv.) for ¹H NMR. The integration of the CH₂Br₂ signal at 4.94 ppm (s, 2H) was compared to the signal of **38** at 7.13 ppm (t, 2H).

**R_{f}** = 0.44 (EtOAc in pentane = 10%, v/v (UV)).

**HRMS-El(m/z)** calc'd for C₉H₅O₁F₇ [M]⁺, 262.0223; found, 269.0223; deviation: -0.1 ppm. **Note:** Due to the volatility of the product the compound was not isolated. Signal at 5.30 ppm corresponds to CH₂Cl₂; signals at 2.34 ppm, 1.68 ppm and 1.08 ppm correspond to *n*-PrCN.

### 3-Fluoro-2,6-dimethylpyridine (39)

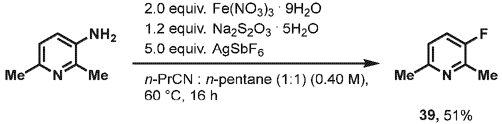

Under an ambient atmosphere, to a 4-mL borosilicate vial equipped with a teflon-coated magnetic stir bar were added 3-amino-2,6-dimethylpyridine (61.1 mg, 0.500 mmol, 1.00 equiv.), Na₂S₂O₃·5H₂O (149 mg, 0.600 mmol, 1.20 equiv.), AgSbF₆ (859 mg, 2.50 mmol, 5.00 equiv.) and iron (III) nitrate nonahydrate (404 mg, 1.00 mmol, 2.00 equiv.) respectively. Then, butyronitrile (n-PrCN) (0.625 mL), *n-*pentane (0.625 mL) (0.4 M overall conc.) were added, the vial was quickly sealed with a septum cap and the reaction mixture stirred at 60 °C for 16 h. After cooling to 23 °C, the resulting mixture was passed through a pad of celite (~2.5 g) by eluting with CH₂Cl₂ (50 ml). Then, the solvent was removed under reduced pressure. The yield was determined by both ¹H NMR spectroscopy at 500 MHz dissolving the residue of the reaction mixture in 0.5 mL of CDCl₃, and using CH₂Br₂ as internal standard (34.9 µL, 86.9 mg, 0.50 mmol, 1.0 equiv.) for ¹H NMR. The integration of the CH₂Br₂ signal at 4.93 ppm (s, 2H) was compared to the signal of **39** at 7.65 (dd, *J* = 8.8, 4.5 Hz, 1H).

**R*_{f}*** = 0.62 (CH₂Cl₂ in MeOH = 10%, v/v (UV)).

**HRMS-El(m/z)** calc'd for C₇H₈N₁F₁ [M]⁺, 125.0635; found, 125.0636; deviation: -1.1 ppm. **Note:** Due to the volatility of the product the compound was not isolated. Signal at 5.29 ppm corresponds to CH₂Cl₂; signals at 2.42 ppm, 1.71 ppm and 1.06 ppm correspond to n-PrCN.

### 4'-Chloro-2-fluoro-1,1'-biphenyl (40)

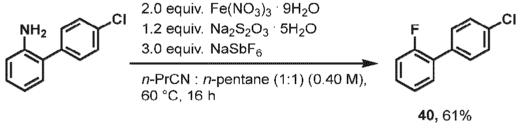

Under an ambient atmosphere, to a 4-mL borosilicate vial equipped with a teflon-coated magnetic stir bar were added 4'-chloro-(1,1'-biphenyl)-2-amine (102 mg, 0.500 mmol, 1.00 equiv.), Na₂S₂O₃·5H₂O (149 mg, 0.600 mmol, 1.20 equiv.), NaSbF₆ (388 mg, 1.50 mmol, 3.00 equiv.) and iron (III) nitrate nonahydrate (404 mg, 1.00 mmol, 2.00 equiv.). Then, butyronitrile (n-PrCN) (0.625 mL), n-pentane (0.625 mL) (0.4 M overall conc.) were added, the vial was quickly sealed with a septum cap and the reaction mixture stirred at 60 °C for 16 h. After cooling to 23 °C, the resulting mixture was passed through a pad of celite (~2.5 g) by eluting with ethyl acetate (50 ml). Then, the solvent was removed under reduced pressure. The residue was purified by column chromatography on silica with pentane as eluent, to give the desired fluoroarene **40** as a colorless liquid (63 mg, 0.305 mmol, 61%). **R*f*** = 0.67 (in pentane (UV)).

**HRMS-EI (m/z)** calc'd for C₁₂H₈Cl₁F₁ [M]⁺, 206.0293; found, 206.0293; deviation: -0.1 ppm.

### Lenalidomide-derived fluoroarene 41

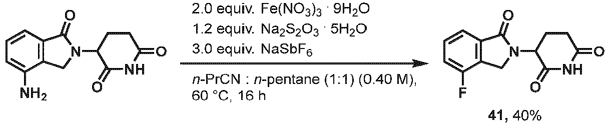

Under an ambient atmosphere, to a 4-mL borosilicate vial equipped with a teflon-coated magnetic stir bar were added Lenalidomide (130 mg, 0.500 mmol, 1.00 equiv.), Na₂S₂O₃·5H₂O (149 mg, 0.600 mmol, 1.20 equiv.), NaSbF₆ (388 mg, 1.50 mmol, 3.00 equiv.) and iron (III) nitrate nonahydrate (404 mg, 1.00 mmol, 2.00 equiv.). Then, butyronitrile (n-PrCN) (1.25 mL) (c = 0.4 M) were added, the vial was quickly sealed with a septum cap and the reaction mixture stirred at 60 °C for 16 h. After cooling to 23 °C, the resulting mixture was passed through a pad of celite (~2.5 g) by eluting with ethyl acetate (50 ml). Then, the solvent was removed under reduced pressure. The residue was purified by column chromatography on silica with ethyl acetate in pentane (60%, v/v) as eluent, to give the desired fluoroarene **41** as a colorless liquid (52 mg, 0.198 mmol, 40%).

**R*f*** = 0.37 (EtOAc in pentane = 80%, v/v (UV)).

**HRMS-ESI (m/z)** calc'd for C₁₃H₁₁F₁N₂Na₁O₃ [M+Na]⁺, 285.0646; found, 285.0650; deviation: -1.7 ppm.

### Aniline Scope for Deaminative Cyanation

### Darunavir-derived benzonitrile 42

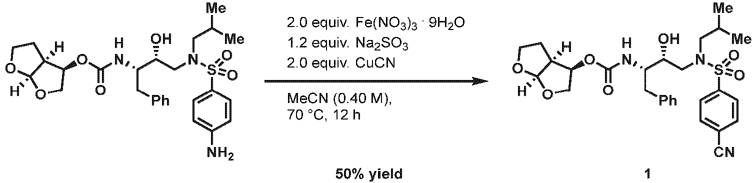

Under an ambient atmosphere, to a 4-mL borosilicate vial equipped with a Teflon-coated magnetic stir bar were added Darunavir (274 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.40 M) was added. The vessel was sealed with a septum cap and heated at 70°C for 12 h in an aluminum block on a heating plate. After cooling to 23 °C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (40:60 (v/v)) to afford 139 mg (50%) of the title compound **42** as light yellow solid.

**R*_{f}*** = 0.72 (EtOAc in pentane = 70%, v/v (UV)).

**HRMS-ESI(m/z)** calc'd for C₂₈H₃₅O₇N₃Na₁S₁⁺ [M+Na]⁺, 580.2088; found, 580.2087; deviation: -0.0 ppm.

### 2-Methyl-3-nitrobenzonitrile (43)

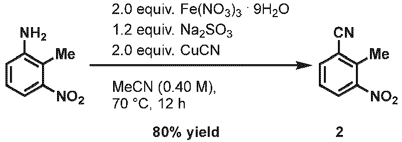

Under an ambient atmosphere, to a 4-mL borosilicate vial equipped with a Teflon-coated magnetic stir bar were added 2-methyl-3-nitroaniline (76.1 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23 °C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (5:95 (v/v)) to afford 65 mg (80%) of the title compound **43** as off white solid.

**R*_{f}*** = 0.31 (EtOAc in pentane = 10%, v/v (UV)).

**HRMS-EI(m/z)** calc'd for C₈H₆N₂O₂⁺ [M]⁺, 162.0424; found, 162.0425; deviation: +1.1 ppm.

### 2-Chloroterephthalonitrile (44)

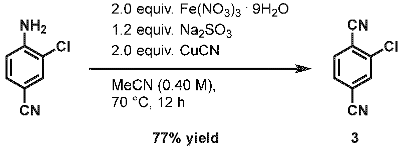

Under an ambient atmosphere, to a 4-mL borosilicate vial equipped with a Teflon-coated magnetic stir bar were added 4-amino-2-chlorbenzonitrile (76.3 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23°C, the resulting mixture was passed through a pad of celite by eluting with ethyl acetate (50 mL).

Then, the solvent was evaporated by rotary evaporation under reduced pressure. The residue was purified by silica gel chromatography by eluting with EtOAc/pentane (5:95 (v/v)) to afford 63 mg (77%) of the title compound **44** as off white solid.

**R*_{f}*** = 0.28 (EtOAc in pentane = 10%, v/v (UV)).

**HRMS-EI(m/z)** calc'd for C₈H₃N₂Cl₁⁺ [M]⁺, 161.9980; found, 161.9979; deviation: +0.3 ppm.

### 2-(Trifluoromethylthio)benzonitrile (45)

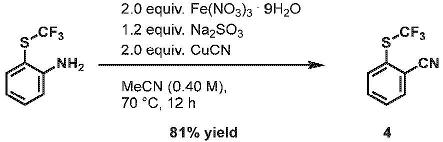

Under an ambient atmosphere, to a 4-mL borosilicate vial equipped with a Teflon-coated magnetic stir bar were added 2-(trifluormethylthio)aniline (96.6 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23°C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (4:96 (v/v)) to afford 82 mg (81%) of the title compound **45** as orange colored oil.

**R*_{f}*** = 0.30 (EtOAc in pentane = 10%, v/v (UV)).

**HRMS-EI(m/z)** calc'd for C₈H₄N₁S₁F₃⁺ [M]⁺,203.0012; found, 203.0011; deviation: +0.1 ppm.

### 6-Bromo-5-methylnicotinonitrile (46)

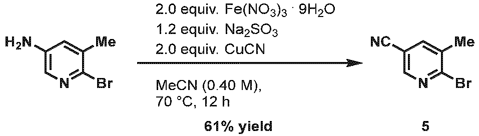

Under an ambient atmosphere, to a 4-mL borosilicate vial equipped with a Teflon-coated magnetic stir bar were added 5-amino-2-brom-3-methylpyridine (93.6 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23°C, the resulting mixture was passed through a pad of celite by eluting with ethyl acetate (50 mL).

Then, the solvent was evaporated by rotary evaporation under reduced pressure. The residue was purified by silica gel chromatography by eluting with EtOAc/pentane (3:97 (v/v)) to afford 60 mg (61%) of the title compound **46** as off white solid.

**R*_{f}*** = 0.37 (EtOAc in pentane = 21%, v/v (UV)).

**HRMS-EI(m/z)** calc'd for C₇H₅N₂Br₁⁺ [M]⁺, 195.9630; found, 195.9631; deviation: +0.5 ppm.

### 2,2-Difluorobenzo[d][1,3]dioxole-5-carbonitrile (47)

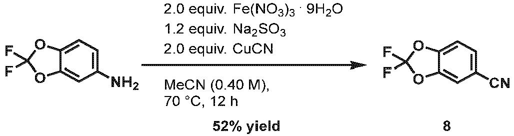

Under an ambient atmosphere, to a 4-mL borosilicate vial equipped with a Teflon-coated magnetic stir bar were added 2,2-Difluorbenzo[d][1,3]dioxol-5-amine (86.6 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23°C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (1:99 (v/v)) to afford 95 mg (52%) of the title compound **47** as a light yellow solid.

**R*_{f}*** = 0.53 (EtOAc in pentane = 10%, v/v (UV)).

**HRMS-EI(m/z)** calc'd for C₈H₃N₁O₂F₂⁺ [M]⁺,183.0126; found, 183.0125; deviation: -0.5 ppm.

### 2,6-Dimethylterephthalonitrile (48)

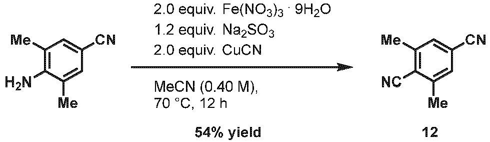

Under an ambient atmosphere, to a 4-mL borosilicate vial equipped with a Teflon-coated magnetic stir bar were added 4-amino-3,5-dimethylbenzonitrile (73.1 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23°C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (2:98 (v/v)) to afford 42 mg (54%) of the title compound **48** as off white solid.

**R*_{f}*** = 0.37 (EtOAc in pentane = 10%, v/v (UV)).

**HRMS-EI(m/z)** calc'd for C₁₀H₈N₂+ [M]⁺, 128.0369; found, 128.0368; deviation: -0.8 ppm.

### Flutamide-derived benzonitrile 49

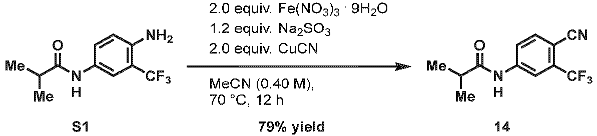

Under an ambient atmosphere, to a 4-mL borosilicate vial equipped with a Teflon-coated magnetic stir bar were added reduced Flutamide S1 (123 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23 °C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (25:75 (v/v)) to afford 101 mg (79%) of the title compound **49** as yellow colored solid.

**R*_{f}*** = 0.41 (EtOAc in pentane = 40%, v/v (UV)).

**HRMS-EI(m/z)** calc'd for C₁₂H₁₁N₂O₁F₃⁺ [M]⁺, 256.0817; found, 256.0814; deviation: -1.5 ppm.

### Ethyl 4-cyanobenzoate (50)

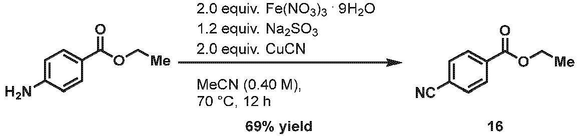

Under an ambient atmosphere, to a 4-mL borosilicate vial equipped with a Teflon-coated magnetic stir bar were added ethyl-4-aminobenzoate (82.6 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23 °C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (1:99 (v/v)) to afford 60 mg (69%) of the title compound **50** as orange colored solid.

**R*_{f}*** = 0.58 (EtOAc in pentane = 10%, v/v (UV)).

**HRMS-EI(m/z)** calc'd for C₇H₂N₁Br₃⁺ [M]⁺, 175.0627; found, 175.0625; deviation: +1.4 ppm.

### 4-(1,1,1,3,3,3-Hexafluoro-2-hydroxypropan-2-yl)benzonitrile (51)

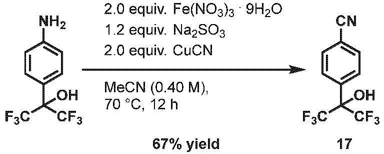

Under an ambient atmosphere, to a 4-mL borosilicate vial equipped with a Teflon-coated magnetic stir bar were added 2-(4-aminophenyl)-1,1,1,3,3,3-hexafluor-2-propanol (130 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23 °C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (2:98 (v/v)) to afford 90 mg (67%) of the title compound **51** as off white solid. **R_{f}** = 0.27 (EtOAc in pentane = 10%, v/v (UV)).

**HRMS-EI(m/z)** calc'd for C₁₀H₅N_{1O}1F₆⁺ [M]⁺, 269.0269; found, 269.0270; deviation: +0.0 ppm.

### rac-Aminoglutethimide-derived benzonitrile rac-52

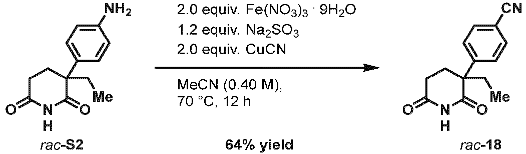

Under an ambient atmosphere, to a 4-mL borosilicate vial equipped with a Teflon-coated magnetic stir bar were added Aminoglutethimide (rac-S2) (116 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23 °C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (25:75 (v/v)) to afford 78 mg (64%) of the title compound rac-**52** as off white solid.

**R*_{f}*** = 0.31 (EtOAc in pentane = 40%, v/v (UV)).

**HRMS-EI(m/z)** calc'd for C₁₄H₁₄N₂O₂ [M]⁺, 242.1049; found, 242.1050; deviation: -0.3 ppm.

### Methyl 3-cyanothiophene-2-carboxylate (53)

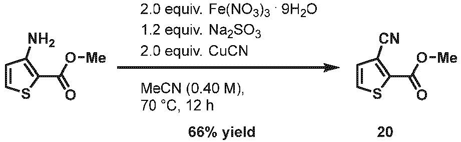

Under an ambient atmosphere, to a 4-mL borosilicate vial equipped with a Teflon-coated magnetic stir bar were added methyl 3-amino-2-thiophenecarboxylate (78.6 mg, 0.500 mmol, 1.00 equiv.), Na₂SO₃ (75.7 mg, 0.600 mmol, 2.00 equiv.), CuCN (89.6 mg, 1.00 mmol, 2.00 equiv.) and iron (III) nitrate nonahydrate (404 mg, 1.00 mmol, 2.00 equiv.). Then, acetonitrile (MeCN) (1.25 mL, c = 0.4 M) was added. The vessel was sealed with a septum cap and heated at 70°C for 12 h in an aluminum block on a heating plate. After cooling to 23 °C, the resulting mixture was passed through a pad of celite by eluting with ethyl acetate (50 mL). Then, the solvent was evaporated by rotary evaporation under reduced pressure. The residue was purified by silica gel chromatography by eluting with EtOAc/pentane (10:90 (v/v)) to afford 55 mg (66%) of the title compound **53** as off white solid.

**R*_{f}*** = 0.25 (EtOAc in pentane = 20%, v/v (UV)).

**HRMS-EI(m/z)** calc'd for C₇H₅N₁O₂S₁⁺ [M]⁺, 167.0035; found, 167.0037; deviation: +1.4 ppm.

### Summary

As detailed above, the present invention provides a process for preparing arylsulfonic acids and derivatives under mild conditions from aryldiazonium salts, though conventional methods often require isolation or accumulation of these potentially hazardous intermediates. Herein the inventors have shown that iron nitrate reduction at 25 °C enables the in situ generation of diazonium salts, which allows direct deaminative chlorosulfonylation, deaminative fluorination, and deaminative cyanation from anilines and primary amino heterocycles via aryl or heteroaryl diazonium salts as fleeting intermediates. Other sulfonic acid derivatives, such as sulfonamides, sulfonyl fluorides, and sulfonic acids are readily accessible from this method.

In conclusion, the inventors have demonstrated that inexpensive Fe(NO₃)₃·9H₂O can generate NO₂ through nitrate reduction with thiosulfate at room temperature (25 °C) which enables the use of the nitrate reduction strategy for the in situ generation of aryl diazonium salts below 85 °C. The iron nitrate reduction was utilized for the deaminative chlorosulfonylation of a variety of structurally and electronically diverse anilines and amino heterocycles, giving access to multiple sulfur-based functional groups directly from the corresponding aromatic amines, without accumulation of the respective diazonium salt. The deaminative fluorination was presented as an additional application of the Fe-mediated nitrate reduction. The inventors expect that the iron nitrate reduction will also enable other deaminative functionalization reactions that require close to ambient temperature. Additionally, the inventors have described a deaminative sulfonic acid synthesis directly from anilines as a previously unknown transformation. Furthermore, the inventors demonstrate that iron nitrate can be reduced with several other sulfur oxygen based reductants and that deaminative cyanation can be carried out as an application.

## Claims

1. Process for the functionalization of an aromatic or heteroaromatic primary amino compound wherein an aromatic or heteroaromatic primary amino compound is reacted with a compound pair of Fe(lll) nitrate and a sulfur oxygen reductant or mixtures thereof, whereby an aromatic or heteroaromatic-N₂⁺-intermediate compound is provided, which is in situ further subjected to a reaction step selected from a sulfochlorination step, a fluorination step or a cyanation step.

2. Process for the functionalization of an aromatic or heteroaromatic primary amino compound according to claim 1, wherein the sulfur-oxygen reductant is selected from alkali or ammonium salts of thiosulfuric acid, thiosulfates, polythionates, dithionates, trithionates, tetrathionates, dithionites, sulfites, bisulfites, sulfur dioxide, or metabisulfites, or mixtures thereof.

3. Process for the functionalization of an aromatic or heteroaromatic primary amino compound according to any one of the preceding claims, wherein the aromatic or heteroaromatiic primary amino compound is selected from C₅ to C₂₀ aryl or heteroaryl primary amino compounds.

4. Process for the functionalization of an aromatic or heteroaromatic primary amino compound according to any one of the preceding claims, wherein the reaction is carried out in an optionally aqueous polar aprotic solvent such as, but not limited to acetonitrile, butyronitrile, ethylacetate, chloroform, 1,2-dichloroethane, dichloromethane, acetone, or mixtures thereof.

5. Process for the functionalization of an aromatic or heteroaromatic primary amino compound according to any one of the preceding claims wherein said aromatic or heteroaromatic-N₂⁺-intermediate compound is reacted with SO₂ in the presence of a Cu(I) or Cu (II) salt or complex and a chloride source whereby a chlorosulfonylated aromatic or heteroaromatic compound is obtained.

6. Process for the functionalization of an aromatic or heteroaromatic primary amino compound according to any one of claims 1 to 4 wherein said aromatic or heteroaromatic-N₂⁺-intermediate compound is reacted with a fluorination agent whereby a fluorinated aromatic or heteroaromatic compound is obtained.

7. Process for the functionalization of an aromatic or heteroaromatic primary amino compound according to claim 6, wherein the fluorination agent is selected from metal salts such as a silver, an ammonium or an alkali salt of tetrafluoric acid, hexafluorophosphoric acid or hexafluoric antimonic acid.

8. Process for the functionalization of an aromatic or heteroaromatic primary amino compound according to any one of the preceding claims wherein said aromatic or heteroaromatic-N₂⁺-intermediate compound may be further reacted in a cyanation step with a Cu(I) or Cu (II) salt or complex and a cyanide source whereby a cyanated aromatic or heteroaromatic compound is obtained.

9. Use of a compound pair of Fe(III) nitrate and a sulfur oxygen reductant or mixtures thereof for the functionalization of an aromatic or heteroaromatic primary amino compound.

10. Use according to claim 9 wherein the sulfur-oxygen reductant is selected from alkali or ammonium salts of thiosulfuric acid, thiosulfates, polythionates, dithionates, trithionates, tetrathionates, dithionites sulfites, bisulfites, sulfur dioxide, or metabisulfites, or mixtures thereof.
